# EUROPEAN PATENT APPLICATION

(11) **EP 1 916 003 A1**
(43) Date of publication of application: **30.04.2008**
(21) Application number: 06090198.0
(22) Date of filing: 25.10.2006
(51) Int. Cl.: A61K 49/08, A61K 49/10, C07H 19/06, A61K 51/04, A61P 35/00, A61K 31/7064

(54) **Thymidine analogs for imaging**

(71) Applicant: Bayer Schering Pharma Aktiengesellschaft, 13353 Berlin (DE)
(72) Inventor: Srinivasan, Ananth, 10629 Berlin (DE); Lehmann, Lutz, 13357 Berlin (DE); Voigtmann, Ulrike, 10115 BERLIN (DE); Berndt, Mathias, 12163 Berlin (DE); Graham, Keith, 10115 Berlin (DE); Zitzmann, Sabine, 10551 Berlin (DE); Fitzner, Ansgar, 20255 Hamburg (DE)

(57) **Abstract**

The present invention relates to nucleoside analogues and more particularly to labelled nucleoside analogues of formula (I). It has been found new thymidine analogues can be stably labelled with a detectable label moiety. Further the present invention relates to methods of making above compounds and use of such compounds for diagnostic imaging of tumor cells and I or treatment of proliferative diseases. In addition, the present invention relates to the preparation and use of positron emitting compounds for positron emission tomography (PET). A kit is also disclosed.

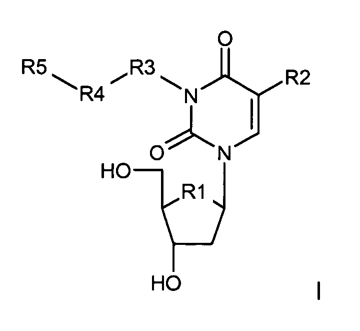

## Description

### FIELD OF INVENTION

The present invention relates to nucleoside analogues and more particularly to labeled nucleoside analogues. It has been found new thymidine analogues can be stably labeled with a detectable label moiety. Further the present invention relates to methods of making above compounds and use of such compounds for diagnostic imaging of tumor cells and / or treatment of proliferative diseases. In addition, the present invention relates to the preparation and use of positron emitting compounds for positron emission tomography (PET).

### BACKGROUND ART

Imaging and measuring proliferation in vivo is a very attractive target for both pre-clinical research and oncological practice, because it could offer the possibility of differentiating between benign and malignant tissues, measuring tumor aggressiveness and evaluating early response to therapy.

The S-phase is the portion of the cell cycle during which DNA replication takes place. Expression of genes related to DNA replication is maximal during early S-phase. In contrast to quiescent cells, proliferating cells synthesize more DNA during the S-phase of the cell cycle. Since radiolabeled nucleoside analogues mimic physiological nucleosides in term of cellular uptake and metabolism through the salvage pathway, they can be used as S-phase marker. Said radiolabeled nucleosides analogues can be activated in vivo either/or by both thymidine kinase (TK) and by thymidylate synthetase (TS) and incorporated to the DNA chain of the cell in DNA duplication phase.
Human cytosolic thymidine Kinase (TK1) has the most stringent substrate specificity among all nucleoside kinases allowing only phosphorylation of native thymidine/deoxyuridine Urd and, to a limited extent, analogues with minor modifications either at the 5-position (Cl, Br, I) or at the 3'-position. (F, N₃). Only recently it was discovered that TK1 also tolerates bulky substituents at the N-3 position, which has been exploited successfully in the design and synthesis of boronated Thd analogues for boron neutron capture therapy (BNCT), an experimental binary radiochemotherapeutic methods for cancer treatment (J Med Chem, 1999. 42(17): p. 3378-89, Bioorg Med Chem., 2005; 13: 161-9, J Med Chem., 2002; 45: 4018-28, Mini Rev Med Chem., 2004; 4: 341-50)

Abe et al. (Eur J Nucl Med 1983;8:258-61 ) reported tumor uptake of 5-¹⁸F-fluoro-2'-deoxyuridine in which the 5-methyl group has been replaced by ¹⁸F in AH109A tumor bearing rats. 5-fluoro-2'-deoxyuridine undergoes cleaving of the C-N glycoside bond to generate 5-fluorouracil and several ¹⁸F soluble metabolites. It was therefore concluded that image represents a mixture of soluble ¹⁸F metabolites, DNA and RNA.
In analogous manner, Conti et al. (Nucl Med Biol. 1994 Nov;21 (8):1045-51) attempted PET imaging of tissue proliferation with ¹¹C thymidine (TdR) labeled at the 5-methyl-position. Rapid degradation of ¹¹C thymidine to metabolites including thymine, dihydrothymine, beta-ureidoisobutyric acid, and beta-aminoisobutyric acid from plasma and tissue extracts was observed. Further short half-life of ¹¹C renders ¹¹C-thymidine unsuitable for tissue proliferation imaging.
Hence, it was recognized that there is a clear need for a nucleoside tracers that are stable in the body, transported and incorporated into DNA.

Shields et al. (Mol Imaging Biol. 2006 May-Jun;B(3):141-50) listed known thymidine radiolabeled positions such as 3'-[F-18]fluoro-3'-deoxythymidine (FLT), 1-(2-deoxy-2-fluoro-D-arabinofuranosyl)uracil (FAU), 1-(2-deoxy-2-fluoro-Darabinofuranosyl)-5-methyluracil (FMAU), 1-(2-deoxy-2-fluoro-D-arabinofuranosyl)-5-bromouracil (FBAU), and 1-(2-deoxy-2-fluoro-D-arabinofuranosyl)-5-iodouracil (FIAU). FLT, FMAU and FIAU can be phosphorylated by the cytosolic form of mammalian thymidine kinase (TK1), while FIAU is preferentially phosphorylated by the herpes simplex virus thymidine kinase (HSV-TK), rather than mammalian TK1. Since FLT lacks the hydroxyl group at 3'-position the 5'-monophosphate is trapped in the cell. Lack of incorporation into DNA does not give true information about DNA proliferation.

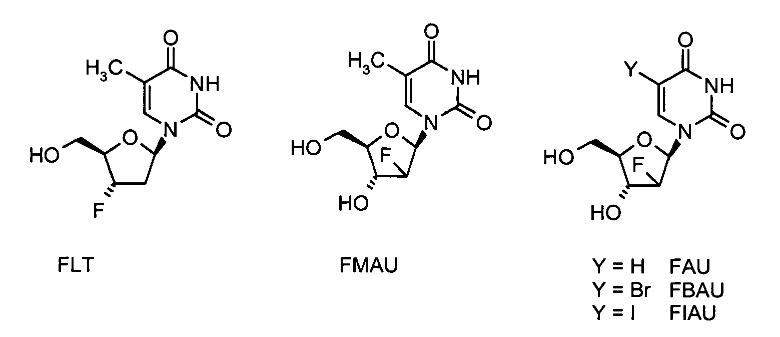

This was confirmed by Been et al. (Eur J Nucl Med Mol Imaging. 2004 Dec;31 (12):1659-72) reporting that ¹⁸F-fluoro-3'-deoxy-3'-flurothymidine (¹⁸F-FLT) uptake in the cell does not reflect true tumour cell proliferation rate because FLT is not incorporated into the DNA. Therefore, ¹⁸F-FLT is more of a measurement of TK1 activity as opposed to specific DNA synthesis.

Gudjonsson et al. (Nucl Med Biol 2001;28:59-65) reported subsequent investigation towards this goal centered upon 5-substituted-⁷⁶Br- 2'-deoxyuridines as possible imaging agents for tissue proliferation. It was theorized that the similarities of ⁷⁶Br at position 5- of deoxyuridine is similar to that of methyl group of thymidine. Even though the uptake of radioactivity was higher in the tumours than in brain parenchyma, only an average of 9% of the radioactivity was found in the DNA fraction. Hence it cannot be used for assessment of proliferation potential.
Haihao Sun et al. (J Nucl Med. 2005 Feb;46(2):292-6) reported that ¹⁸F-1-(2'-deoxy-2'-fluoro-β-D-arabinofuranosyl)thymidine (FMAU) is selectively retained in DNA of the proliferating tissues and is resistant to degradation. FMAU shows high uptake in other tissues such as heart, kidney and liver tissues. But the uptake into cells is too low for sufficient signal intensity. Hence, there is a need of efficient imaging agent targeting selectively cell proliferation.

Toyohara et al. (Nuclear Medicine and Biology, 2006: 33: 751-764) compared in vitro derivative of 2'-deoxyuridine containing a fluoroalkyl groups (i.e. methyl and ethyl group) at the C5 position to derivative thymidine containing a fluoroalkyl groups (i.e. methyl, ethyl and propyl group) at the N3-position. The latter derivatives are phosphorylated by recombinant thymidine kinase 1 (TK-1), are potent substrate of a mouse erythrocyte transporter and are not degraded by recombinant Escherichia coli thymidine phosphorylase.
Further Toyohara et al. (Nuclear Medicine and Biology, 2006: 33: 765-772) reported that despite N³-(2-[¹⁸F]fluroethyl)-thymidine acceptable properties in vitro, the said compound is not a suitable ligand for imaging tumor cell proliferation

CA 2 252 144 A1 discloses a dual acting anti-tumor drug comprising a moiety of butanoyl- or retinoyl-groups and a hydroxy- containing anti-tumor group such as a nucleoside analogs connected to one another at the position 3' of the sugar moiety. The disclosed compounds have a complex structure.

Hence there is a clear need for stable and efficient marker being also useful for treatment of proliferative diseases. Indeed true radiolabelled tracers are needed. Such tracer should not only be substrates for human cytosolic TK1 but also should have the ability to be incorporated into DNA by virtue of the presence of 3'-hydroxy group so as to render true measurement of DNA proliferation.
It has been surprisingly found that the compounds of the invention are transported into the cell and are activated by thymidine kinase and/or thymidylate synthetase enzymes then converted to the corresponding triphosphates, followed by incorporation into DNA.
This phenomenon produces images which corresponds to true measure of tissue proliferation.

### SUMMARY OF THE INVENTION

The present invention relates to nucleoside analogues and more particularly to labeled nucleoside analogues. It has been found new thymidine analogues that can be labeled with a detectable label moiety. In a preferred embodiment, the thymidine analogue will contain a positron emitting moiety. In addition the current invention provides method for the preparation of detectable thymidine analogues useful for imaging as well as for therapeutic applications.

The invention relates also to kit comprising new thymidine analogues.

Accordingly, it is the object of the present invention to provide compounds, compositions, and methods to identify susceptible tumors in biopsy specimens or via external imaging, and/or inhibit or reduce the replication or spread of tumor cells.
It is an object of the present invention to provide compounds and methods useful for external imaging applications. In preferred embodiments, the invention includes the selection, preparation, and uses of nucleosides labeled with fluorine-18 (¹⁸F), a positron emitter.
It is another object of the present invention to provide a treatment for tumors and other diseases characterized by abnormal cell proliferation by administrating these compounds or compositions either alone or in combination with other agents that inhibit tumor growth and/or with other classes of therapeutics used to treat such diseases.

It is another object of the present invention to assess the impact of other treatments (e.g., by radiotherapy or other drugs) upon tumor growth and to monitor the efficacy of supportive treatments. In preferred embodiments the supportive treatments may be bone marrow transplant and/or stimulation by growth factors. In other preferred embodiments, the present invention may be used to monitor and assess the course of liver regeneration after surgery or injury. In preferred embodiments, the treatments will be drugs intended to inhibit thymidylate synthetase. The methods of the present invention permit treatment individualization using surrogate markers such as external imaging. Other embodiments of the invention may be useful in selecting the most effective drugs to be used against tumors in humans.

It is an object of the present invention to provide a method for monitoring the expression of genes introduced in gene therapy applications.

Other features and advantages of the present invention will be apparent from the following description of preferred embodiments.

### DETAILED DESCRIPTION

The present invention provides compounds, compositions, and methods for diagnosing and/or treating proliferative diseases. The compounds of the present invention include nucleoside analogues which are activated by thymidine kinase (TK) and/or thymidylate synthetase (TS) enzymes in an effective amount for diagnosis or to reduce or inhibit the replication or spread of tumor cells.

In a first aspect, the present invention is directed to a compound of Formula I 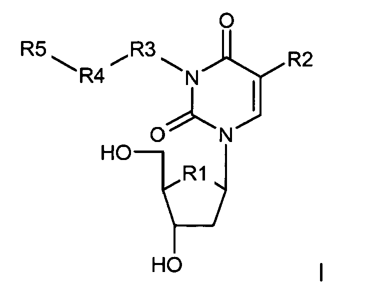 wherein
- R1: is O, S, CH₂, C(=CH₂), C=O, C=S, NH or N-alkyl;
- R2: is H, linear or branched alkyl, CF₃, Cl, Br or I;
- R3: is selected from
a) [alkoxyl]ₙ-alkyl;
b) higher alkyl;
c) benzoyl-alkyl; and
d) benzoyl
   wherein n is 1 to 10;
- R4: is a linker;
- R5: is a radioisotope label
and pharmaceutically acceptable salt, hydrate or solvate thereof.

In preferred embodiments of compounds of Formula I, R3 is a benzoyl. In a further preferred embodiment R3 is higher alkyl. More preferably the higher alkyl is a C8 to C18 alkyl chain. More preferably the higher alkyl is a C₈ to C₁₂ alkyl chain. The alkyl chain being preferably linear.

In a further preferred embodiment R3 is [alkoxyl]ₙ-alkyl wherein n is 1 to 10. More preferably [alkoxyl]ₙ-alkyl is selected from
i. -(CH₂-CH₂-O)ₘ-(CH₂-CH₂) -;
ii. -(CH₂-CH₂-CH₂O)-(CH₂-CH₂-O)ₘ-(CH₂-CH₂)-; and
iii. -(CH₂-CH₂-CH₂O)-(CH₂-CH₂-O)ₘ-(CH₂-CH₂-CH₂)-wherein m is 1 to 6.

In a further preferred embodiment R3 is benzoyl-alkyl.
Each preferred embodiment of R3 can be combined to each other wherein R3 refers to 1 to 3 listed embodiments.

In preferred embodiments of compounds of Formula I, R1 is selected from O, S and C(=CH₂).

In preferred embodiments of compounds of Formula I, R2 is a C₁ to C₄ alkyl.
More preferably R2 is CH₃.

In preferred embodiments of compounds of Formula I, R4 is defined as a bond or Aryl-L
wherein L is selected from
a) -C(O)N(H);
b) -C((O)N(Me);
c) -SO₂-N(H)-; and
d) -SO₂-N(Me)-;
   and wherein Aryl is an aromatic moiety optionally substituted with:
   a) Hydrogen;
   b) Halo;
   c) Cyano;
   d) Nitro;
   e) Trifluoromethyl;
   f) -S(O)₂-methyl;
   g) -S(O)₂-ethyl;
   h) -C(O)-Me or
   a combination thereof.

In a more preferred embodiment the aromatic moiety is a phenyl.

In a more preferred embodiment, L is selected from -C(O)N(H) and -SO₂-N(Me)-.

In preferred embodiments of compounds of Formula I, the radioisotope label R5 is selected from ¹⁸F, ⁷⁷Br, ⁷⁶Br, ¹²³I, ¹²⁵I, and ¹¹C. In a more preferred embodiment, the radioisotope label R5 is ¹⁸F.

A preferred series of compounds of Formula I include derivatives having the following structures:
N³-(3-(N-(4-[¹⁸F]-fluoro-3-cyano-benzoyl))aminopropyl)-thymidine 13aa,
N³-(3-(N-(4-[¹⁸F]-fluoro-3-trifluoromethyl-benzoyl))aminopropyl)-thymidine 13bb,
N³-(3-(N-(4-[¹⁸F]-fluoro-2-chloro-benzoyl))aminopropyl)-thymidine 13cc,
N³-(2-(2-[¹⁸F]Fluoroethoxy)-ethyl)-*O*,*O*-bis-(1'-methoxy-1'-cyclohexyl) thymidine 15,
N³-(3-(N-(4-[¹⁸F]-fluoro-3-cyano-benzoyl))aminopropyl)-thymidine 16,
N³-(2-(2-[¹⁸F]Fluoroethoxy)-ethyl)-thymidine 20,
N³-(3-(N-(4-[¹⁸F]-fluorobenzoyl))amino-propyl)- thymidine.

Thymidine analogues labeled with positron emitting atoms such as ¹⁸F are more useful for imaging.

In a second aspect, the invention is directed to a compound of formula (II) 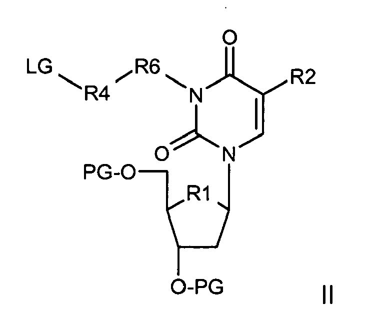 wherein
PG is selected from:
a) hydrogen;
b) SiR⁷₃;
c) CH₂-Z;
d) C(O)-Q;
e) C(O)O-E;
f) -(R⁸-phenyl);
g) -((R⁸)₂-phenyl);
h) tetrahydropyranyl;
i) (1-alkoxy)-alkyl;
j) (1-alkoxy)-cycloalkyl;
k) allyl; and
l) *tert*-butyl;
Z is selected from
a) phenyl;
b) alkoxy;
c) benzyloxy;
d) triphenyl;
e) (methoxyphenyl)phenyl;
f) di(methoxyphenyl)phenyl;
g) α-naphtyldiphenyl;
h) hydrogen; and
i) methylsulfanyl;
Q is selected from
a) hydrogen;
b) C₁-C₅ linear or branched alkyl;
c) halomethyl;
d) dihalomethyl;
e) trihalomethyl;
f) phenyl;
g) biphenyl;
h) triphenylmethoxymethyl; and
i) phenoxymethyl;
E is selected from
a) lower linear or branched alkyl;
b) methoxymethyl;
c) vinyl;
d) allyl;
e) benzyl;
f) methoxyphenyl;
g) dimethoxyphenyl; and
h) nitrophenyl;
i) phenyl;
R⁷ is independently from each other selected from
a) lower linear or branched alkyl;
b) phenyl; and
c) benzyl;
   R⁸ is selected from methoxy and halo;
   LG is a leaving group;
   R1, R2 and R4 are defined as above for formula I; and
   R6 is selected from

a) [alkoxyl]ₙ-alkyl
b) C₁-C₁₈ alkyl;
c) benzoyl-alkyl; and
d) benzoyl;
wherein n is 1 to 10
and pharmaceutically acceptable salt, hydrate or solvate thereof.

In preferred embodiments of compounds of Formula II, PG is selected from a group comprising
a) hydrogen
b) CH₂-Z
c) (2-tetrahydropyranyl)
d) (1-alkoxy)-cycloalkyl
e) allyl and
f) *tert*-butyl.
In further preferred embodiments of compounds of Formula II, PG is selected from a group comprising
a) hydrogen
b) CH₂-Z
c) tetrahydropyranyl
d) (1-alkoxy)-cycloalkyl
e) *tert*-butyl and
f) (1-alkoxy)-cycloalkyl.
In a more preferred embodiments of compounds of Formula II, PG is (1-alkoxy)-cycloalkyl. More preferably (1-alkoxy)-cycloalkyl is (1-methoxy)cyclohexyl.

In preferred embodiments of compounds of Formula II, Z is selected from a group comprising
a) phenyl
b) alkoxy
c) benzyloxy
d) triphenyl and
e) (methoxyphenyl)phenyl.
In further preferred embodiments of compounds of Formula II, Z is selected from a group comprising
a) alkoxy and
b) benzyloxy.
More preferably alkoxy is methoxy.
In preferred embodiments of compounds of Formula II, Q is selected from a group comprising
a) hydrogen
b) C₁-C₅ linear or branched alkyl
c) phenyl and
d) phenoxymethyl.
In further preferred embodiments of compounds of Formula II, Q is selected from a group comprising
a) C₁-C₅ linear or branched alkyl and
b) phenyl.
More preferably C₁-C₅ linear or branched alkyl is methyl.

In preferred embodiments of compounds of Formula II, E is selected from a group comprising
a) lower linear or branched alkyl
b) methoxymethyl and
c) phenyl.
In further preferred embodiments of compounds of Formula II, E is selected from a group comprising
a) lower linear or branched alkyl and
b) phenyl.
More preferably lower linear or branched alkyl is methyl or tert-butyl.

In preferred embodiments of compounds of Formula II, R⁷ is independently from each other selected from a group comprising
a) lower linear or branched alkyl wherein alkyl is selected from methyl, ethyl, isopropyl, and tert-butyl
b) phenyl and
c) benzyl.
In further preferred embodiments of compounds of Formula II, R⁷ is independently from each other selected from a group comprising
a) methyl
b) ethyl
c) isopropyl
d) tert-butyl and
e) phenyl.

In preferred embodiments of compounds of Formula II, the leaving group (LG) is selected from
1. NO₂, (CH₃)₃N⁺ if R4 is aryl for radiofluorination;
2. (alkyl)₃Sn if R4 is aryl for radioiodination and ¹¹C-methyliodide (Stille-cross-coupling), and
3. Cl, Br , I, OTs, OMs, OTf, ONs if R4 is bond for radiofluorination.
4. hydroxy if R4 is aryl for ¹¹C alkylation.

In preferred embodiments of compounds of Formula II, R6 is a benzoyl.
In a further preferred embodiment R6 is a C1 to C7 alkyl chain or C8 to C18 alkyl chain. More preferably R6 is independently C1 to C6 or C8 to C12 alkyl chain. More preferably R6 is C8 to C18 alkyl chain.
In a further preferred embodiment R6 is [alkoxyl]ₙ-alkyl wherein n is 1 to 10.
More preferably [alkoxyl]ₙ-alkyl is selected from
a) -(CH₂-CH₂-O)ₘ-(CH₂-CH₂)-;
b) -(CH₂-CH₂-CH₂O)-(CH₂-CH₂-O)ₘ-(CH₂-CH₂)-; and
c) -(CH₂-CH₂-CH₂O)-(CH₂-CH₂-O)ₘ-(CH₂-CH₂-CH₂)-wherein m is 1 to 6.
In a further preferred embodiment R6 is benzoyl-alkyl.
Each preferred embodiment of R6 can be combined to each other wherein R6 refers to 1 to 3 embodiments.

R1, R2 and R4 preferred embodiments disclosed above for compounds of formula I apply also for compounds of formula II.

A preferred series of compounds of Formula II include derivatives having the following structures:
3-t-butyldimethylsilyloxybutyl-4-(1-methoxy-cyclohexyloxy)-5(1-methoxy-cyclohexyloxy-methyl)-thymidine 2a,
3-t-butyldimethylsilyloxyhexyl-4-(1-methoxy-cyclohexyloxy)-5(1-methoxy-cyclohexyloxy-methyl)-thymidine 2b,
3-(4-Hydroxybutyl)-4'-(1-methoxy-cyclohexyloxy)-5'(1-methoxy-cyclohexyloxymethyl)-thymidine 3a,
3-(6-Hydroxyhexyl)-4'-(1-methoxy-cyclohexyloxy)-5'-(1-methoxy-cyclohexyloxymethyl)-thymidine 3b ,
3-(4-O-Tosyl)butyl-4'-(1-methoxy-cyclohexyloxy)-5'-(1-methoxy-cyclohexyloxymethyl)-thymidine 4a,
3-(6-O-Tosyl)hexyl)-4'-(1-methoxy-cyclohexyloxy)-5'-(1-methoxy-cyclohexyloxymethyl)-thymidine 4b,
3-(4-O-Tosyl)butyl-thymidine 5a,
3-(6-O-Tosyl)hexyl-thymidine 5b,
3-(4-O-methanesulfonyl)butyl-4'-(1-methoxy-cyclohexyloxy)-5'-(1-methoxy-cyclohexyloxymethyl)-thymidine 6a,
3-(6-O-methanesulfonyl))hexyl)-4'-(1-methoxy-cyclohexyloxy)-5'-(1-methoxy-cyclohexyloxymethyl)-thymidine 6b,
3-(4-O-methanesulfonyl)butyl-thymidine 7a,
3-(6-O-methanesulfonyl)hexyl-thymidine 7b,
3-(3-(4-trimethylaminobenzoyl)amino)propyl-4'-(1-methoxy-cyclohexyloxy)-5'-(1-methoxy-cyclohexyloxymethyl)-thymidine 10 and
3-(3-(4-trimethylaminobenzoyl)amino)propyl-thymidine 11.

In a third aspect, the invention is directed to the use of compounds of formula I as diagnostic imaging agent. Optionally compounds of formula II a precursor of compounds of formula I can be used instead of compound of formula I. In other words, the invention is directed to the use of a compound of formula I for the manufacture of diagnostic imaging agent.
In a preferred embodiment the use of compounds of formula I is directed to the use of compounds of formula I as diagnostic imaging agent for positron emission tomography (PET). The diagnostic imaging agent can be used for imaging tissue proliferations, hyperplastic inflammation, benign tumours or malignant tumours.
In a preferred embodiment the above disclosed use applies to compound abstained below and named compound of formula III.

Additionally, the third aspect of the present invention is directed to a method for diagnosing tissue proliferations, hyperplastic inflammation, benign tumours or malignant tumours comprising the steps of
a) administering to a patient a compound of formula I, and
b) measuring positron emission by PET.
   In a preferred embodiment, the diagnosis is a diagnosis concerning localization, progress or determination of therapeutic effect of tissues proliferation, hyperplastic inflammation or benign or malignant tumours.

In a fourth aspect, the invention is directed to the use of compounds of formula I as medicament. Optionally compounds of formula II are precursor of compounds of formula I. In other words, the invention is directed to the use of a compound of formula I for the manufacture of medicament. Said medicament can be used for treating proliferative diseases wherein proliferative disease is a disease developing malignant tumor selected from malignant lymphoma, pharyngeal cancer, lung cancer, liver cancer, bladder tumor, rectal cancer, prostatic cancer, uterine cancer, ovarian cancer, breast cancer, brain tumor, and malignant melanoma.

In a fifth aspect, the invention is directed to a composition comprising a compound of formula I or II and a pharmaceutical acceptable carrier or diluent.

In a sixth aspect, the invention is directed to methods for obtaining compound of formula (III) 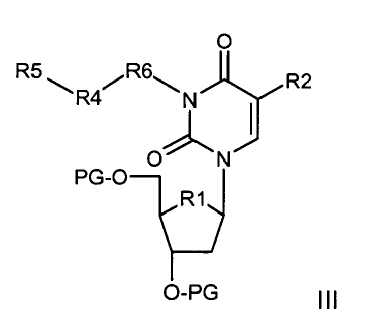 R1, R2, R4, R5 and R6 are as defined above.
Surprisingly 2 methods have been identified for obtaining compounds of formula III. The first method consists of a straight forward radioisotope labeling reaction i.e. one-step method. The second method consists of substitution of the secondary nitrogen of the thymine moiety of a compounds of formula V using a radiolabeled compound of formula IV as substituent.

The first method comprises the step of reacting compound of formula (II) 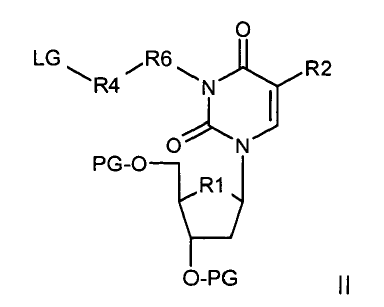 wherein
PG , LG, R1, R2, and R4 are as defined above
R6 is selected from
a) [alkoxyl]ₙ-alkyl
b) C₁-C₁₈ alkyl;
c) benzoyl-alkyl and
d) benzoyl
wherein n is 1 to 10;
with R5 the radioisotope label as defined above,
optionally thereafter the compound of formula III can be converted into a pharmaceutically acceptable salt, hydrate or solvate thereof.

In a preferred embodiment of the first method (one-step method) the reaction is a radiofluorination. Because of the short half life of ¹⁸F (110 minutes) the fluorinated nucleoside must be prepared on the day of its clinical use. In the circumstances, the reactions steps are optimized for short time with yield as a secondary consideration. The one step method is characterized by reacting a compound of Formula II with an appropriate radiofluorination agent. The reagents, solvents and conditions which can be used for this radiofluorination are common and well-known to the skilled person in the field. See e.g. J. Fluorine Chem. 27 (1985) 117-191.
In a preferred method of preparing a compound of Formula III, the step of radiofluorination of a compound of Formula II is carried out at a temperature selected from a range from 40°C to 120°C.

In a more preferred method of preparing a compound of Formula III, the step of radiofluorination of a compound of Formula II is carried out at a temperature selected from a range from 60°C to 100°C.

The second method comprises the steps of coupling compound of formula (IV) 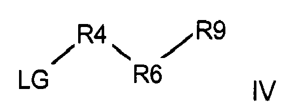 wherein
LG is a leaving group,
R9 is selected from iodo, bromo, chloro, mesyloxy, tosyloxy, trifluormethylsulfonyloxy and nonafluorobutylsulfonyloxy,
R4 is defined as above,
R6 is selected from
a) [alkoxyl]ₙ-alkyl
b) C₁-C₁₈ alkyl;
c) benzoyl-alkyl and
d) benzoyl
wherein n is 1 to 10
with R5 that is defined as a radioisotope label (see above), for obtaining a single radiolabeled compound (IV), then reacting the resultant labeled compound (IV) with a compound of formula (V) 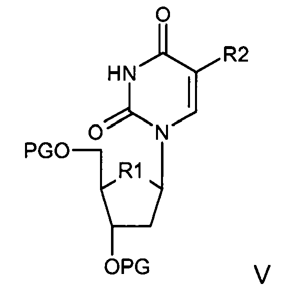 wherein PG, R1 and R2 are defined as above,
optionally thereafter the compound of formula III can be converted into a pharmaceutically acceptable salt, hydrate or solvate thereof.

In preferred embodiment of both methods R6 is C₈-C₁₈ alkyl.

In preferred embodiments of both methods, the radioisotope label R5 is selected from ¹⁸F, ⁷⁷Br , ⁷⁶Br, ¹²³I, ¹²⁵I , and ¹¹C. In a more preferred embodiment, the radioisotope label R5 is ¹⁸F.

In preferred embodiments of first method and second method disclosed above the compound of formula III is a compound of formula I.

In a seventh aspect, the present invention is directed to a kit comprising
a) compound of formula I;
b) compound of formula II;
c) compound of formula III; or
d) compounds of formula IV and V.

The kit comprising one or more of the above listed compounds is useful for imaging tissue proliferations, hyperplastic inflammation, benign tumours or malignant tumours.
The kit will allow user to obtain compound of Formula I or III ready to use for imaging or treatment. Within the kit one of the above coupling reaction may take place i.e. compound of Formula II reacting with a radio isotope label or compound of Formula V coupled to compound of Formula IV.

A further aspect of the invention is directed to following compounds:
N³-(3-(N-(4-[¹⁹F]-fluorobenzoyl))amino-propyl)-*O*,*O*-bis-(1'-methoxy-1'-cyclohexyl) thymidine 12,
N³-(3-(N-(4-[¹⁹F]-fluorobenzoyl))amino-propyl)-thymidine 13,
N³-(3-(N-(3-cyano-4-[¹⁹F]-fluorobenzoyl))amino-propyl)-*O*,*O*-bis-(1'-methoxy-1'-cyclohexyl) thymidine 12a,
N³-(3-(N-(3-trifluoromethyl-4-[¹⁹F]-fluorobenzoyl))amino-propyl)-*O*,*O*-bis-(1'-methoxy-1'-cyclohexyl) thymidine 12b,
N³-(3-(N-(2-chloro-4-[¹⁹F]-fluorobenzoyl))amino-propyl)-*O*, *O*-bis-(1'-methoxy-1'-cyclohexyl) thymidine 12c,
N³-(3-(N-(3-cyano-4-[¹⁹F]-fluorobenzoyl))amino-propyl)-thymidine 13a,
N³-(3-(N-(4-[¹⁹F]-fluoro-3-trifluormethyl-benzoyl))amino-propyl)-thymidine 13b,
N³-(3-(N-(2-chloro-4-[¹⁹F]-fluorobenzoyl))amino-propyl)-thymidine 13c,
3-(4-[¹⁹F]-Fluorobutyl)-thymidine 18,
3-(6-[¹⁹F]-Fluorohexyl)-thymidine 19,
3-(4-[¹⁸F]-Fluorobutyl)-thymidine 14, and
3-(6-[¹⁸F]-Fluorohexyl)-thymidine 17.

As used in the specification and appended claims, unless specified to the contrary, the following terms have the meaning indicated:
The term "alkyl" refers to a linear or branched chain monovalent or divalent radical consisting of solely carbon and hydrogen, containing no unsaturation and having from one to eight carbon atoms, such as methyl, ethyl, n-propyl, 1-methlyethyl (iso-propyl), n-butyl, n-pentyl, n-heptyl and the like. "C₈ to C₁₈ alkyl" refers to a alkyl chain having from eight to eighteen carbon atoms, such as octyl, nonyl, decyl, undecyl, dodecyl and the like. More preferably "C₈ to C₁₈ alkyl" refers to a linear alkyl chain.
The term "lower alkyl chain" refers to a linear or branched alkyl chain of C₁ to
C₆ carbons.
The term "cycloalkyl" refers to monocyclic ring of 3 to 15 carbon atoms selected from but not limited to cyclopropyl, cyclobutyl, cyclopentyl, cyclohexyl, cycloheptyl, cyclooctyl, cyclononyl, decahydro-naphthalenyl or cyclodecyl or bicyclic ring of 6 to 20 carbon atoms wherein at least one of the ring comprises a minimum of 5 carbon atoms.
The term "[alkyoxyl]-alkyl" refers to a radical of the formula [Ra-O]-Ra- wherein each Ra is an lower alkyl radical as defined above.
The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.
The term halo refers to fluorine (F), chlorine (Cl), bromine (Br), and iodine (I).
The term "allyl" refers to an alkene hydrocarbon group with the formula H₂C=CH-CH₂- such as H₂C=CH-CH₂OH.
The term "vinyl" refers to organic compound containing CH₂=CH- group.

Unless otherwise specified, when referring, to the compounds of formula I, II and III per se as well as to any pharmaceutical composition thereof the present invention includes all of the salts, hydrates, solvates, complexes, and prodrugs of the compounds of the invention. Prodrugs are any covalently bonded compounds, which releases the active parent pharmaceutical according to formula I or III. "Pharmaceutical acceptable salt" includes acid.
"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, propionic acid, pyruvic acid, maleic acid, malonic acid, succinic acid, fumaric acid, tartaric acid, citric acid, benzoic acid, mandelic acid,methanesulfonic acid, ethanesulfonic acid, p-toluenesulfonic acid, salicylic acid, trifluoroacetic acid and the like.

The present invention includes all derivative compounds that would be in the scope of the invention and obvious for the skilled person. Consequently the present invention includes other halogen or non halogen isotopes such as ¹⁹F, ⁷⁵Br , ⁷⁶Br , ⁷⁹Br, ¹²³I,¹²⁴I,¹²⁷I,¹³¹I, ¹³N or ^{34m}Cl. Further the proposed methods of the invention can also be used to introduce other halogens or radioactive isotopes thereof, e.g. ¹⁹F, ⁷⁵Br, ⁷⁶Br, ⁷⁹Sr, ¹²³I, ¹²⁴I, ¹²⁷I, ¹³¹I, ¹³N or ^{34m}Cl.

The present invention provides compositions comprising compounds of Formula I, II or III and a pharmaceutical acceptable carrier or diluent. The composition is a pharmaceutically acceptable composition or formulation obtained by methods well known to those of ordinary skill in the art. The composition can be administered by standard routes. More preferably the composition is administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11^{th} ed. In general, when used to treat cell proliferative disorders, the dosage of the invention compounds will depend on the type of tumour, condition being treated, the particular compound being utilized, and other clinical factors such as weight, condition of the human or animal, and the route of administration. It's to be understood that the present invention has application for both human and veterinarian use.

In accordance with the invention, the radiolabeled compounds according to Formula I or III either as a neutral composition or as a salt with a pharmaceutically acceptable counter-ion are administered in a single unit injectable dose. Any of the common carriers known to those with skill in the art, such as sterile saline solution or plasma, can be utilized after radiolabelling for preparing the injectable solution to diagnostically image various organs, tumors and the like in accordance with the invention. Such techniques include the step of bringing into association the active ingredient and the pharmaceutical carrier(s) or diluent(s). In general the formulations are prepared by uniformly and intimately bringing into association the active ingredient with the liquid carrier.
Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

### EXAMPLES

Unless defined otherwise, all technical and scientific terms used herein have the same meaning as commonly understood by one of the ordinary skill in the art to which this invention belongs. Although any methods and materials similar or equivalent to those described herein can be used in the practice or testing of the present invention, the preferred methods and materials are described below. The following schematic examples relates to the preparation of a compounds according to Formula I or III using a compound according to Formula II, IV and V. The methods presented as schemes below are in principle suitable to generate compounds over the whole breadth of Formula I or III using compounds over the whole breadth of Formula II, IV and V. The examples presented below are given merely to illustrate a way of labelling a compound according to Formula II or V to arrive at a compound according to Formula I or III and is not to be understood as to limit the invention to the methods exemplified herein.

### 1. Synthesis of compound of Formula II:

The compound of Formula II is a compound that may have both hydroxyl group of the furan moiety protected by a protecting group (PG) and the N3- pyrimidine is substituted with a leaving group (LG). Protected or non-protected thymidine analogue can be used as starting material.
The starting material is synthesized as followed:

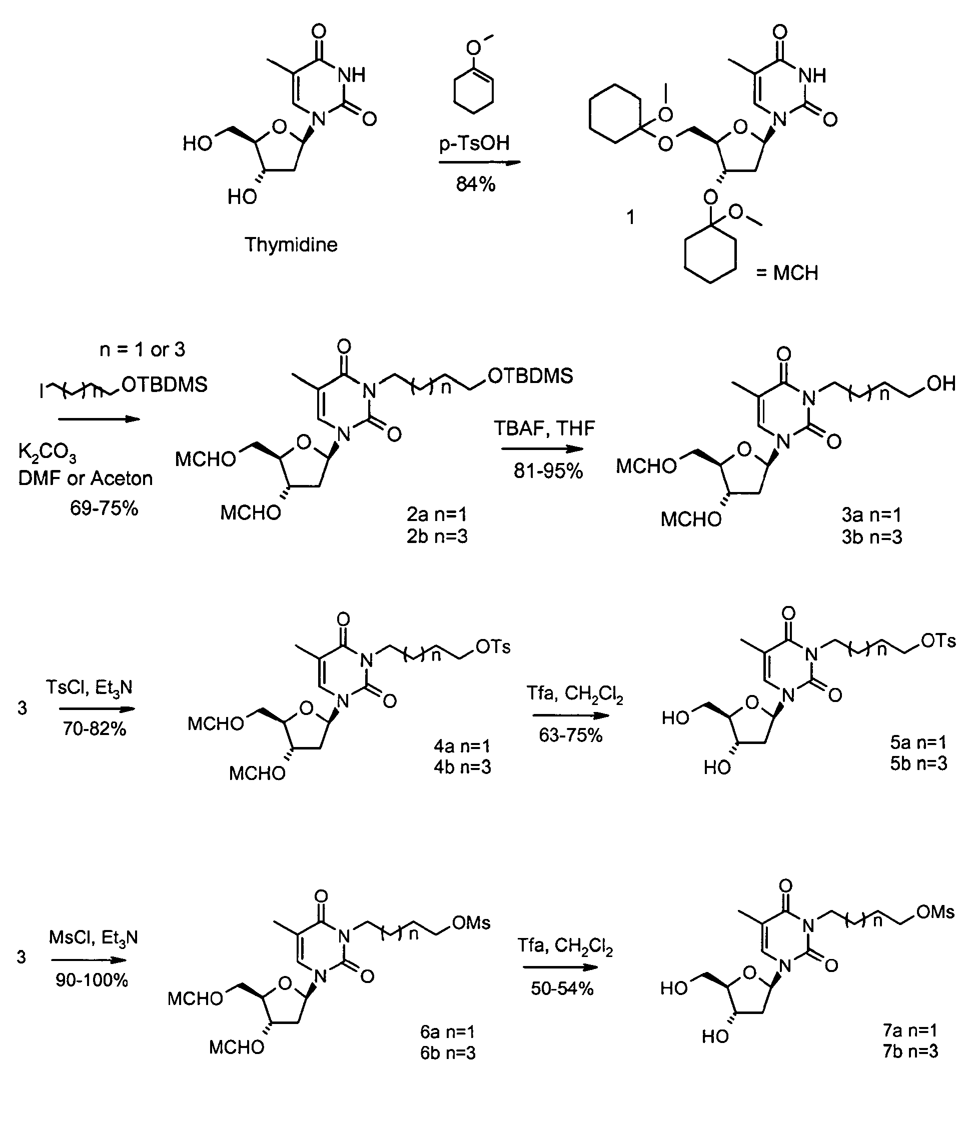

### 1.1 Synthesis of 4-(1-methoxy-cyclohexyloxy)-5-(1-methoxy-cyclohexyloxymethyl)-thymidine (1):

10 g (41.28 mmol) Thymidine, 39.3 g (115.62 mmol) methyl cyclohexene and 250 mg (1.31 mmol) p-toluol sulfonic acid were solved in 500 ml dichloromethane and stirred at rt for 72h. Then the reaction mixture was diluted with dichloromethane, washed with bicarbonate and brine and dried over sodium sulphate. After filtration and removal of the solvent the crude product was purified by chromatography on silica gel to give 13.75 g (71%) of compound 1.
¹H-NMR (d-6 DMSO): δ = 7.46 (s, 1 H), 6.11 (t, 1H), 4.38 (m, 1 H), 3.95 (m, 1 H), 3.46 (m,2H), 3.06 (s, 3H), 3.05 (s, 3H), 2.13 (m, 2H), 1.75 (s, 3H), 1.72-1.20 (m, 20H) ppm.

### 1.2 Synthesis of 3-t-butyldimethylsilyloxyalkyl-4-(1-methoxy-cyclohexyloxy)-5(1-methoxy-cyclohexyloxy-methyl)-thymidine (2)

(2a) 3-t-butyldimethylsilyloxybutyl-4-(1-methoxy-cyclohexyloxy)-5(1-methoxy-cyclohexyloxy-methyl)-thymidine
(2b) 3-t-butyldimethylsilyloxyhexyl-4-(1-methoxy-cyclohexyloxy)-5(1-methoxy-cyclohexyloxy-methyl)-thymidine
1 (2.14 mmol) was solved in 10 ml DMF/acetone 1:1 and treated with (5.79 mmol) potassium carbonate. To this mixture (2.79 mmol) of the iodo building block solved in 2 ml DMF/acetone was added. The reaction mixture was stirred at 50°C for 3 h and then at rt over night, filtered and concentrated. The residue was taken up in water and extracted with dichloromethane. The combined organic phases were washed with brine, dried over sodium sulphate and concentrated. Finally, the crude material was purified by chromatography on silica gel to give 69-75% of 2.
2a: ¹H-NMR (d-6 DMSO): δ = 7.51 (s, 1 H), 6.14 (t, 1 H), 4.38 (m, 1 H), 3.97 (m, 1 H), 3.77 (t, 2H), 3.53 (t, 2H), 3.47 (m, 2H), 3.06 (s, 3H), 3.04 (s, 3H), 2.18 (m, 2H), 1.80 (s, 3H), 1.75-1.20 (m, 24H), 0.80 (s, 9H), 0.03 (s, 6H) ppm.
2b: ¹H-NMR (d-6 DMSO): δ = 7.51 (s, 1 H), 6.15 (t, 1 H), 4.39 (m, 1 H), 3.98 (m, 1 H), 3.74 (t, 2H), 3.51 (t, 2H), 3.47 (m, 2H), 3.06 (s, 3H), 3.04 (s, 3H), 2.18 (m, 2H), 1.80 (s, 3H), 1.75-1.09 (m, 28H), 0.81 (s, 9H), 0.03 (s, 6H) ppm.

### 1.3 Synthesis of 3-(4-Hydroxyalkyl)-4'-(1-methoxy-cyclohexyloxy)-5'(1-methoxy-cyclohexyloxymethyl)-thymidine (3)

3-(4-Hydroxybutyl)-4'-(1-methoxy-cyclohexyloxy)-5'(1-methoxy-cyclohexyloxymethyl)-thymidine 3a,
3-(6-Hydroxyhexyl)-4'-(1-methoxy-cyclohexyloxy)-5'-(1-methoxy-cyclohexyloxymethyl)-thymidine 3b

Compound 2 (8.12 mmol) was solved in 300 ml THF, cooled down to 0°C and treated with (16 mmol) TBAF 1 mol in THF. The reaction mixture was stirred at rt for 4 h, diluted with ethyl acetate, washed with water, dried over sodium sulphate, filtered and concentrated. The residue was purified by chromatography on silica gel to give 81-95% of compound 3.
3a: ¹H-NMR (d-6 DMSO): δ = 7.52 (s, 1 H), 6.16 (t, 1 H), 4.38 (m, 1 H), 4.35 (t, 1 H), 3.97 (m, 1 H), 3.77 (t, 2H), 3.47 (m, 2H), 3.33 (m, 2H), 3.06 (s, 3H), 3.04 (s, 3H), 2.18 (m, 2H), 1.80 (s, 3H), 1.75-1.20 (m, 24H) ppm.
3b: ¹H-NMR (d-6 DMSO): δ = 7.51 (s, 1 H), 6.15 (t, 1 H), 4.39 (m, 1 H), 4.30 (t, 1 H), 3.98 (m, 1 H), 3.73 (t, 2H), 3.47 (m, 2H), 3.33 (m, 2H), 3.06 (s, 3H), 3.04 (s, 3H), 2.18 (m, 2H), 1.80 (s, 3H), 1.75-1.09 (m, 28H) ppm.

### 1.4 Synthesis of 3-(4-O-Tosyl)alkyl-4'-(1-methoxy-cyclohexyloxy)-5'-(1-methoxy-cyclohexyloxymethyl)-thymidine (4)

3-(4-O-Tosyl)butyl -4'-(1-methoxy-cyclohexyloxy)-5'-(1-methoxy-cyclohexyloxymethyl)-thymidine 4a,
3-(6-O-Tosyl)hexyl)-4'-(1-methoxy-cyclohexyloxy)-5'-(1-methoxy-cyclohexyloxymethyl)-thymidine 4b
Starting from 3a and 3b.

Compound 3 (0.44 mmol) was solved in 5.7 ml dichloromethane, cooled down to 0°C and then treated with (0.88 mmol) triethyl amine and (0.49 mmol) p-toluene sulfonyl chloride. The reaction mixture was stirred at rt for 46 h and concentrated. The crude residue was purified by chromatography on silica gel to give 70-82% of compound 4.
4a: ¹H-NMR (d-6 DMSO): δ = 7.75 (d, 2H), 7.51 (s, 1 H), 7.44 (d, 2H), 6.14 (t, 1 H), 4.38 (m, 1 H), 4.00 (t, 2H), 3.96 (m, 1 H), 3.70 (t, 2H), 3.47 (m, 2H), 3.06 (s, 3H), 3.04 (s, 3H), 2.38 (s, 3H), 2.17 (m, 2H), 1.79 (s, 3H), 1.75-1.30 (m, 24H) ppm.
4b: ¹H-NMR (d-6 DMSO): δ = 7.77 (d, 2H), 7.56 (s, 1 H), 7.50 (d, 2H), 6.20 (t, 1 H), 4.44 (m, 1 H), 4.03 (m, 1 H), 4.00 (t, 2H), 3.74 (t, 2H), 3.52 (m, 2H), 3.11 (s, 3H), 3.09 (s, 3H), 2.43 (s, 3H), 2.23 (m, 2H), 1.85 (s, 3H), 1.75-1.09 (m, 28H) ppm.

### 1.5 Synthesis of 3-(4-O-Tosyl)alkyl-thymidine (5)

3-(4-O-Tosyl)butyl-thymidine 5a,
3-(6-O-Tosyl)hexyl-thymidine 5b
Starting from 4a and 4b

Compound 4 (0.1 mmol) was solved in 1 ml dichloromethane, cooled down to 0°C and titrated with a solution of trifluoracetic acid in dichloromethane (10%) until complete conversion of the starting material. The reaction mixture was diluted with dichloromethane, washed with bicarbonate and brine, dried over sodium sulphate, filtrated and concentrated. The residue was purified by chromatography on silica gel to give 63-57% of compound 5.
5a: ¹H-NMR (d-6 DMSO): δ = 7.73 (d, 2H), 7.73 (s, 1 H), 7.44 (d, 2H), 6.15 (t, 1H), 5.22 (d, 1 H), 5.01 (t, 1 H), 4.20 (m, 1 H), 3.98 (t, 2H), 3.74 (m, 1 H), 3.69 (t, 2H), 3.52 (m, 2H), 2.38 (s, 3H), 2.06 (m, 2H), 1.77 (s, 3H), 1.48 (m, 4H) ppm.
5b: ¹H-NMR (d-6 DMSO): δ = 7.73 (d, 2H), 7.72 (s, 1 H), 7.46 (d, 2H), 6.17 (t, 1 H), 5.20 (d, 1 H), 5.00 (t, 1 H), 4.44 (m, 1 H), 4.20 (m, 1 H), 3.96 (t, 2H), 3.74 (m, 2H), 3.69 (t, 2H), 2.38 (s, 3H), 2.05 (m, 2H), 1.77 (s, 3H), 1.50-1.09 (m, 8H) ppm.

### 1.6 Synthesis of 3-(4-O-methanesulfonyl)alkyl-4'-(1-methoxy-cyclohexyloxy)-5'-(1-methoxy-cyclohexyloxymethyl)-thymidine (6)

3-(4-O-methanesulfonyl)butyl -4'-(1-methoxy-cyclohexyloxy)-5'-(1-methoxy-cyclohexyloxymethyl)-thymidine 6a,
3-(6-O-methanesulfonyl))hexyl)-4'-(1-methoxy-cyclohexyloxy)-5'-(1-methoxy-cyclohexyloxymethyl)-thymidine 6b
Starting from 3a and 3b.

Compound 3 (0.18 mmol) was solved in 2.6 ml dichloromethane, cooled down to 0°C and then treated with (1.6 mmol) triethyl amine and (0.26 mmol) methane sulfonyl chloride. The reaction mixture was stirred at rt for 1 h and concentrated. The crude residue was purified by chromatography on silica gel to give 90-100% of compound 6.
6a: ¹H-NMR (d-6 DMSO): δ = 7.53 (s, 1 H), 6.14 (t, 1 H), 4.38 (m, 1 H), 4.16 (t, 2H), 3.98 (m, 1 H), 3.79 (t, 2H), 3.47 (m, 2H), 3.12 (s, 3H), 3.06 (s, 3H), 3.04 (s, 3H), 2.19 (m, 2H), 1.81 (s, 3H), 1.75-1.20 (m, 24H) ppm.
6b: ¹H-NMR (d-6 DMSO): δ = 7.52 (s, 1 H), 6.16 (t, 1 H), 4.38 (m, 1 H), 4.14 (t, 2H), 3.95 (m, 1 H), 3.75 (t, 2H), 3.47 (m, 2H), 3.12 (s, 3H), 3.06 (s, 3H), 3.06 (s, 3H), 2.18 (m, 2H), 1.80 (s, 3H), 1.75-1.09 (m, 28H) ppm.

### 1.7 Synthesis of 3-(4-O-methanesulfonyl)alkyl-thymidine (7)

3-(4-O-methanesulfonyl)butyl-thymidine 7a,
3-(6-O- methanesulfonyl)hexyl-thymidine 7b
Starting from 6a and 6b

Compound 6 (0.1 mmol) was solved in 1 ml dichloromethane, cooled down to 0°C and titrated with a solution of trifluoracetic acid in dichloromethane (10%) until complete conversion of the starting material. The reaction mixture was diluted with dichloromethane, washed with bicarbonate and brine, dried over sodium sulphate, filtrated and concentrated. The residue was purified by chromatography on silica gel to give 50-54% of compound 7.
7a: ¹H-NMR (d-6 DMSO): δ = 7.74 (s, 1 H), 6.17 (t, 1 H), 5.22 (d, 1 H), 5.02 (t, 1 H), 4.20 (m, 1 H), 4.17 (t, 2H), 3.79 (t, 2H), 3.69 (m, 1 H), 3.52 (m, 2H), 3.13 (s, 3H), 2.06 (m, 2H), 1.79 (s, 3H), 1.59 (m, 4H) ppm.
7b: ¹H-NMR (d-6 DMSO): δ = 7.72 (s, 1 H), 6.17 (t, 1 H), 5.21 (d, 1 H), 5.01 (t, 1 H), 4.20 (m, 1 H), 4.14 (m, 1 H), 3.75 (m, 3H), 3.55 (m, 2H), 3.12 (s, 3H), 2.07 (m, 2H), 1.78 (s, 3H), 1.75-1.2 (m, 8H) ppm.

### 1.8 Synthesis of 3-(3-(4-trimethylaminobenzoyl)amino)propyl-4'-(1-methoxy-cyclohexyloxy)-5'-(1-methoxy-cyclohexyloxymethyl)-thymidine (10) and 3-(3-(4-trimethylaminobenzoyl)amino)propyl-thymidine (11)

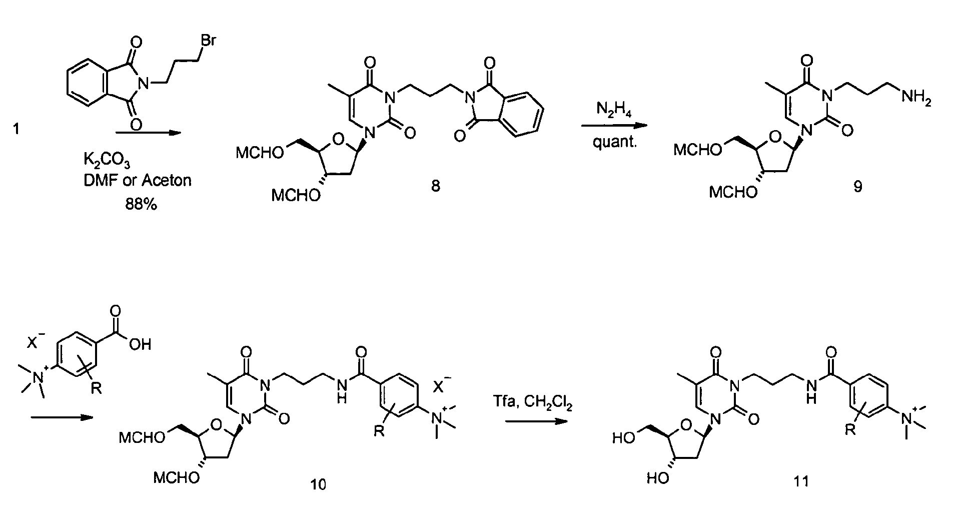

3-(3-pthalimido)propyl -4'-(1-methoxy-cyclohexyloxy)-5'-(1-methoxy-cyclohexyloxymethyl)-thymidine (8):
5 g (10.7 mmol) of compound 1 and 4 g (28.9 mmol) potassium carbonate were solved in 50 ml DMF/acetone 1:1. A solution of 3.74 g (13.9 mmol) N-(3-bromopropyl) pthalimide in 10 ml DMF/acetone 1:1 was added. The reaction mixture was stirred at 50°C for 6 h and further 4 d at rt and filtrated. The precipitate was washed with acetone and the solution was diluted with water and extracted with dichloromethane. The combined organic phases were washed with brine, dried over sodium sulphate, filtrated and concentrated. The residue was purified by chromatography on silica gel to give 5.8 g (83%) of compound 8.
¹H-NMR (d-6 DMSO): δ = 7.82 (m, 4H), 7.50 (s, 1 H), 6.11 (t, 1 H), 4.38 (m, 1H), 3.96 (m, 1 H), 3.79 (t, 2H), 3.55 (t, 2H), 3.47 (m, 2H), 3.06 (s, 3H), 3.04 (s, 3H), 2.16 (m, 2H), 1.84 (m, 2H), 1.75 (s, 3H), 1.74-1.25 (m, 20H) ppm.
3-(3-amino)propyl-4'-(1-methoxy-cyclohexyloxy)-5'-(1-methoxy-cyclohexyloxymethyl)-thymidine (9)
2 g (3.06 mmol) of compound 8 were solved in 80 ml ethanol and treated with 0.4 ml (8.28 mmol) hydrazine hydrate. The reaction mixture was stirred at rt for 4d. The precipitate was filtered off and the remaining solution was concentrated. The crude product 9 1.6 g (100%) was used without further purification.
¹H-NMR (d-6 DMSO): δ = 7.52 (s, 1 H), 6.16 (t, 1H), 4.38 (m, 1 H), 3.96 (m, 1 H), 3.80 (t, 2H), 3.45 (t, 2H), 3.47 (m, 2H), 3.06 (s, 3H), 3.04 (s, 3H), 2.16 (m, 2H), 1.80 (s, 3H), 1.74-1.25 (m, 20H) ppm.

### 2. Synthesis of compound of Formula III using the one-step approach method

### 2.1 Preparation of 3-(4-[¹⁸F]Fluorobutyl)-thymidine from 3-(4-O-methanesulfonyl)butyl-thymidine (14)

[¹⁸F]fluoride (263 MBq) in 200 µl was trapped on a QMA-cartridge (Waters, Sep Pak Light QMA Part.No.: WAT023525) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue mesylate precursor (3.5 mg, 8.9 µmol) in acetonitrile (1.0 ml) was added and the mixture was heated at 100 °C for 15 min. After cooling at room temperature HCl (1 N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 21 MBq of 3-(4-[¹⁸F]Fluorobutyl)-thymidine was collected.
HPLC purification: Agilent 1100 series, radioactivity detector: Raytest Gabi; HPLC Column: Zorbax C18 Bonus-RP; 9.2x250; Solvent A: water +0.1% TFA; Solvent B: acetonitrile:water (9:1) + 0.1 % TFA; Gradient: isocratic 31%B for 2 min then 31% to 41% B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained (3.1 min) witch co-elutes with the reference compound 3-(4-[¹⁹F]Fluorobutyl)-thymidine. The product is identical to the one prepared by the alkylation of thymidine with 4-¹⁸F-fluorobutylbromide described above.

### 2.2 Synthesis of N-3 [¹⁸F]-alkoxyalkyl substituted thymidine analogues using the one-step approach method

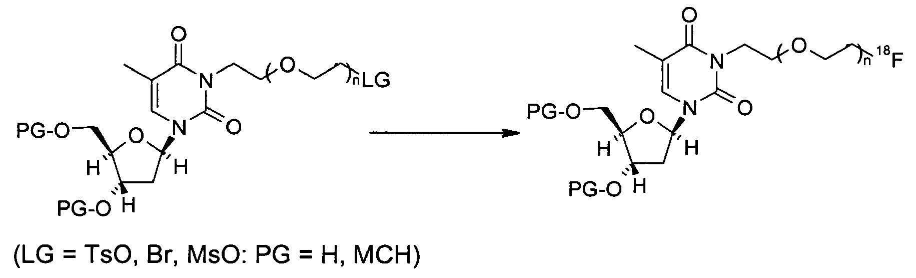

### N³-(2-(2-[¹⁸F]Fluoroethoxy)-ethyl)-O,O-bis-(1'-methoxy-1'-cyclohexyl) thymidine (15) from corresponding mesyloxy precursor

(n=1; LG = mesyloxy and PG=MCH)

[¹⁸F]fluoride (247 MBq) in 200 µl was trapped on a QMA-cartridge (Waters, Sep Pak Light QMA Part.No.: WAT023525) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue precursor XX (3.0 mg) in acetonitrile (1.0 ml) was added and the mixture was heated at 100 °C for 15 min. After cooling at room temperature HCl (1N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 29MBq of the desired product was collected.
HPLC purification: Agilent 1100 series, radioactivity detector: Raytest Gabi; HPLC Column: Zorbax C18 Bonus-RP; 9.2x250; Solvent A: water +0.1% TFA; Solvent B: acetonitrile:water (9:1) + 0.1 % TFA; Gradient: isocratic 31%B for 2 min then 31% to 41% B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained witch co-elutes with the F-19 reference compound. The product is identical to the one prepared by the alkylation described above.

### 2.3 Synthesis of N3 Ar-L substituted thymidine analogues using the one-step approach method

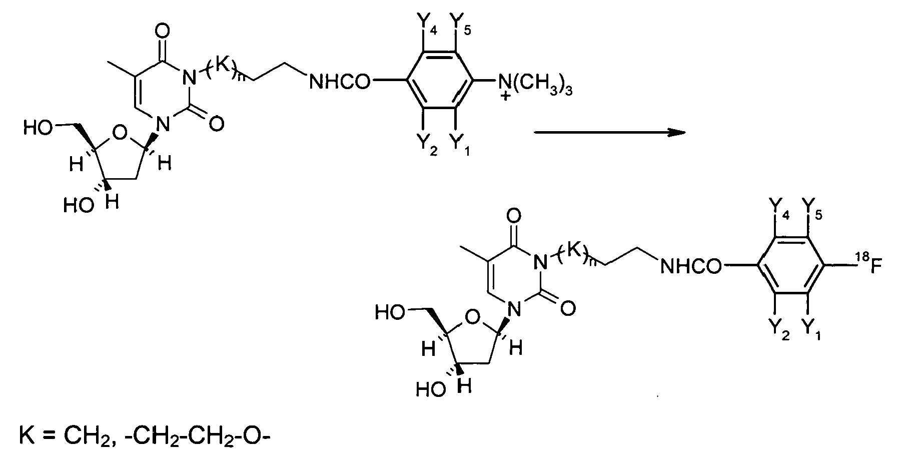

### 2.3.1.

### a) Synthesis of 3-Cyano-4-fluoro-benzoic acid (2.3.1.a).

To a stirred solution of 15.0 g (97,6 mmol) 2-fluoro-5-formyl-benzonitrile (Aldrich), 150 ml dest. water and 630 ml t-butanol are added 40.8 g (361 mmol) sodium chlorite and 35.9 g (230 mmol) sodium hydrogen phosphate dihydrate. The reaction mixture is stirred over night and poured into a diluted aqueous hydrogen chloride solution (pH=3.5). The pH value is readjusted to pH=3.5 by aqueous hydrogen chloride. The aqueous solution is extracted trice with dichloromethane/isopropanol (10:1). The combined organic phases are dried (sodium sulphate) and concentrated. The residue is purified by extraction with sodium hydrogen carbonate solution and dichloromethane, acidification with aqueous solution and subsequent filtering. The solid crude product **2a** is obtained in 90% yield (14.5 g, 87.8 mmol) and is used for the next step without purification.
MS-ESI: 166 (M⁺ +1, 77),
Elementary analysis:

| | |
|---|---|
| Calculated: | C 58.19% H 2.44% F 11.51% N 8.48% |
| Determined: | C 58.81% H 2.42% F 11.41% N 8,47% |

### b) Synthesis or 3-Cyano-4-fluoro-benzoic acid methyl ester (2.3.1. b).

To a stirred suspension of 16.0 g (96,9 mmol) **2.3.1.a** and 161 ml methanol are added 30.4 g (387,6 mmol) acetyl chloride drop wisely at 0°C. The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **2.3.1.b** is obtained in 78,1% yield (13.5 g; 75.7 mmol)
MS-ESI: 180 (M⁺ +1,57),
Elementary analysis:

| | |
|---|---|
| Calculated: | C 60.34% H 3.38% F 10.60% N 7.82% |
| Determined: | C 60.51% H 3.39% F 10.57% N 7,80% |

### c) Synthesis of 3-Cyano-4-dimethylamino-benzoic acid methyl ester (2.3.1.c).

To a stirred solution of 24.0 g (134 mmol) **2.3.1.b** and 240 ml dimethylsulphoxid are added 13.2 g (161 mmol) dimethylamine hydrochloride and 38,9 g (281 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **2.3.1.c** is obtained in 94% yield (25,7 g, 126 mmol) and is used for the next step without purification.
MS-ESI: 205 (M⁺ +1, 59),
Elementary analysis:

| | |
|---|---|
| Calculated: | C 64.69% H 5.92% N 13.72% |
| Determined: | C 64.79% H 5,95% N 13.69% |

### d) Synthesis of (2-Cyano-4-methoxycarbonyl-phenyl)-trimethyl-ammonium trifluoro-methanesulfonate (2.3.1.d).

To a stirred solution of 6.16 g (30.2 mmol) **2.3.1.c** and 110 ml dichloromethane are added 50.0 g (302 mmol) methyltriflate (Aldrich) drop wisely. The reaction mixture is stirred over night and diethylether is added. After evaporation of one third of the solvent volume the desired compound precipitates and the rest of the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **2.3.1.d** is obtained in 69 % yield (20.8 mmol, 7.68 g).
MS-ESI: 219 (M⁺, 100),
Elementary analysis:

| | |
|---|---|
| Calculated: | C 42.39% H 4.10% F 15.47% N 7.61% |
| Determined: | C 42.42% H 4.12% F 15.41% N 7.59% |

### e) Synthesis of Trifluoro-methanesulfonate(4-carboxy-2-cyano-phenyl)-trimethyl-ammonium; (2.3.1.e).

A solution of 4,01 g (10.9 mmol) **2.3.1.d**, 95 ml dest. water and 95 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **2.3.1.e** is obtained in 93% yield (3.59 g, 10.1 mmol) and crude compound **2.3.1.e** is used for the next step without purification.
MS-ESI: 205 (M⁺, 100),
Elementary analysis:

| | |
|---|---|
| Calculated: | C 40.68% H 3.70% F 16.09% N 7.91% |
| Determined: | C 40.72% H 3.71% F 16.06% N 7,91% |

### f) Synthesis of N³-((N'-(4-trimethylammonium-3-cyano-benzoyl)))-3-amino-propyl-thymidine-triflate salt. (2.3.1.f).

To a stirred solution of 0.2 mmol **2.3.1 e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0,2 mmol **9** in 2 ml DMF. The mixture is stirred intensively for 8h. The mixture is evaporated and purified by C-18 RR chromatography (water/acetonitril). The desired product **2.3.1.f** is obtained in 65% yield - 82 mg.
MS-ESI: 486 (M⁺, 100),
Elementary analysis:

| | |
|---|---|
| Calculated: | C 47.24% H 5.07% F 8.97% N 11.02% |
| Determined: | C 47.26% H 5.02% F 9.00% N 11,01 % |

### g) N³-(3-(N-(4-[¹⁸F]-fluoro-3-cyano-benzoyl))aminopropyl)-thymidine 13aa

[¹⁸F]fluoride (276 MBq) in 200 µl was trapped on a QMA-cartridge (Waters, Sep Pak Light QMA Part.No.: WAT023525 ) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue 3.0 mg precursor N³-(3-(N-(4-trimethylammonium-3-cyano-benzoyl))aminopropyl)-thymidine triflate salt (compare 11) in acetonitrile (1.0 ml) was added and the mixture was heated at 70°C for 15 min. After cooling at room temperature HCl (1 N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 29MBq of the desired product 13a was collected.
HPLC purification: Agilent 1100 series, radioactivity detector: Raytest Gabi; HPLC Column: Zorbax C18 Bonus-RP; 9.2x250; Solvent A: water + 0.1 % TFA; Solvent B: acetonitrile:water (9:1) + 0.1 % TFA; Gradient: isocratic 26%B for 2 min then 26% to 41 % B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained witch co-elutes with the F-19 reference compound.

### 2.3.2.

### a) 4-Dimethylamino-3-trifluoromethyl-benzoic acid methyl ester (2.3.2.a)

To a stirred solution of 4,48 g (22.5 mmol) 4-Fluoro-3-trifluoromethyl-benzoic acid methyl ester (Rarechem) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **2.3.2.a** is obtained in 72 % yield (4,00 g, 16,2 mmol).
MS-ESI: 248 (M⁺ +1, 100).

| | |
|---|---|
| Elementary analysis: | C 53.44% H 4.89% F 23.05% N 5.67% |
| Determined: | C 53.48% H 4.90% F 23.03% N 5.65% |

### b) Trifluoro-methanesulfonate(4-methoxycarbonyl-2-trifluoromethyl-phenyl)-trimethyl-ammonium (2.3.2.b)

To a stirred solution of 3.09 g (12.5 mmol) **2.3.2.a** and 50 ml dichloromethane are added 20.5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **2.3.2.b** is obtained in 69% yield (3.55 g, 8,63 mmol).
MS-ESI: 262 (M⁺, 67),
Elementary analysis:

| | |
|---|---|
| Calculated: | C 37.96% H 3.68% F 27.71 % N 3.41% |
| Determined: | C 38.00% H 3.62% F 27.68% N 3.40% |

### c) Trifluoro-methanesulfonate(4-carboxy-2-trifluoromethyl-phenyl)-trimethylammonium (2.3.2.c).

A solution of 2.84 g (6,92 mmol) (**2.3.2.b**), 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 89% yield (2.45 g; 6.16 mmol) and crude compound (**2.3.2.c**) is used for the next step without purification.
MS-ESI: 248 (M⁺, 100),
Elementary analysis:

| | |
|---|---|
| Calculated: | C 36.28% H 3.30% F 28.69% N 3.53% |
| Determined: | C 36.29% H 3.31% F 28.67% N 3.51% |

### d) Synthesis of thymidine derivative N³-((N'-(4-trimethylammonium-3-trifluormethyl-benzoyl)))-3-amino-propyl-thymidine - triflate salt (2.3.2.d)

To a stirred solution of (0.2 mmol) **2.3.2.c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0,2 mmol **9** in 2 ml DMF. The mixture is stirred intensively for 8h. The mixture is evaporated and purified by C-18 RR chromatography (water/acetonitril). The desired product **2.3.2.d** is obtained in 75% yield - 101 mg.
MS-ESI: 529 (M⁺, 100),
Elementary analysis:
Calculated: C 44.25% H 4.75% F 16.80% N 8.26% Determined: C 44.27% H 4.76% F 16.79% N 8,24%

### e) N³-(3-(N-(4-[¹⁸F]-fluoro-3-trifluoromethyl-benzoyl))aminopropyl)-thymidine (13bb)

[¹⁸F]fluoride (276 MBq) in 200 µl was trapped on a QMA-cartridge (Waters, Sep Pak Light QMA Part.No.: WAT023525) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue 3.0 mg precursor **2.3.2.d** in acetonitrile (1.0 ml) was added and the mixture was heated at 70°C for 15 min. After cooling at room temperature HCl (1 N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 29MBq of the desired product 13b was collected.
HPLC purification: Agilent 1100 series, radioactivity detector: Raytest Gabi;
HPLC Column: Zorbax C18 Bonus-RP; 9.2x250; Solvent A: water + 0.1 % TFA; Solvent B: acetonitrile:water (9:1) + 0.1% TFA; Gradient: isocratic 26%B for 2 min then 26% to 41 % B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained witch co-elutes with the F-19 reference compound.

### 2.3.3.

### a) 2-Chloro-4-dimethylamino-benzoic acid methyl ester (2.3.3.a).

To a stirred solution of 4,00g (20.6 mmol) 2-chloro-4-fluoro-benzoic acid methyl ester (Apollo) and 60 ml dimethylsulphoxid are added 2.03 g (24,7 mmol) dimethylamine hydrochloride and 5,97 g (43.2 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **2.3.3.**a is obtained in 99% yield (4,36 g, 20.4 mmol) and is used for the next step without purification.
MS-ESI: 213/215 (M⁺ +1, 64 / 48).
Elementary analysis:

| | |
|---|---|
| Calculated: | C 56.21% H 5.66% N 6.56% |
| Determined: | C 56.39% H 5,67% N 6.54% |

### b) Synthesis of (3-chloro-4-methoxycarbonyl-phenyl)-trimethyl-ammonium trifluoro-methanesulfonate (2.3.3.b)

To a stirred solution of 4.49 g (21.0 mmol) **2.3.3**.a and 75 ml dichloromethane are added 34,5 g (210 mmol) methyltriflate (Aldrich) drop wisely. The reaction mixture is stirred for 2 days at room temperature. 17 g (10 mmol) methyltriflate (Aldrich) are added and the reaction mixture is stirred at 40°C for 20 h. The reaction mixture is cooled to 20°C and diethylether is added. The desired compound precipitates and the solvent is decanted and the solid is washed extensively (ten times) with large amounts of diethylether. The solid is dried by use of oil pump vacuum and purified by (C-18) RP-column chromatography (acetonitril/water - gradient 1:99 to 80:20). The desired compound **2.3.3.**b is obtained in 86% yield (6,86 g, 18,1 mmol).
MS-ESI: 228/230 (M⁺, 81),
Elementary analysis:

| | |
|---|---|
| Calculated: | C 38.15% H 4.00% F 15.09% N 3.71% |
| Determined: | C 38.18% H 4.02% F 15.04% N 3.70% |

### c) Synthesis of (4-carboxy-3-chloro-phenyl)-trimethyl-ammonium trifluoro-methanesulfonate (2.3.3.c).

A solution of 0.5 g (1.32 mmol) **2.3.3.**b, 12 ml dest. water and 12 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude **2.3.3.**c is obtained in 98% yield (471 mg, 1.3 mmol) and crude compound **2.3.3.**c is used for the next step without purification.
MS-ESI: 214/216 (M⁺, 89/56),
Elementary analysis:

| | |
|---|---|
| Calculated: | C 36.32% H 3.60% F 15.67% N 3.85% |
| Determined: | C 36.37% H 3.63% F 15.61% N 3,83% |

### d) Synthesis of thymidine derivative N³-((N'-(4-trimethylammonium-2-chloro)))-3-amino-propyl-thymidine-triflate salt (2.3.3.d)

To a stirred solution of (0.2 mmol) **2.3.3.c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0,2 mmol **9** in 2 ml DMF. The mixture is stirred intensively for 8h. The mixture is evaporated and purified by C-18 RR chromatography (water/acetonitril). The desired product **2.3.2.d** is obtained in 70% yield- 9,03 mg.
MS-ESI: 496 (M⁺, 100),
Elementary analysis:
Calculated: C 44.69% H 5.00% F 8.84% N 8.69% Determined: C 44.73% H 5.02% F 8.84% N 8,68%

### e) N³-(3-(N-(4-[¹⁸F]-fluoro-2-chloro-benzoyl))aminopropyl)-thymidine (13cc)

[¹⁸F]fluoride (276 MBq) in 200 µl was trapped on a QMA-cartridge (Waters, Sep Pak Light QMA Part.No.: WAT023525) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue 3.0 mg precursor **2.3.3.d** in acetonitrile (1.0 ml) was added and the mixture was heated at 70°C for 15 min. After cooling at room temperature HCl (1 N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 29MBq of the desired product 13c was collected.
HPLC purification: Agilent 1100 series, radioactivity detector: Raytest Gabi; HPLC Column: Zorbax C18 Bonus-RP; 9.2x250; Solvent A: water + 0.1 % TFA; Solvent B: acetonitrile:water (9:1) + 0.1% TFA; Gradient: isocratic 26%B for 2 min then 26% to 41 % B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained witch co-elutes with the F-19 reference compound.

### 2.3.4 Synthesis of N³-(3-(N-(4-[¹⁸F]-fluoro-3-cyano-benzoyl))aminopropyl)-thymidine (16)

n=3
Y1, Y2, Y4, = hydrogen; Y5 = cyano

[¹⁸F]fluoride (276 MBq) in 200 µl was trapped on a QMA-cartridge (Waters, Sep Pak Light QMA Part.No.: WAT023525) and eluted using a solution of Kryptofix 2.2.2 (6 mg) and potassium carbonate (1 mg) in aqueous acetonitrile (1.5 ml; 66 % acetonitrile). The solution was dried by addition and evaporation of anhydrous acetonitril (4 x 1 ml) under a gentle nitrogen stream at 120 °C. The reaction was cooled to room temperature and to the dried residue precursor (3.0 mg) in acetonitrile (1.0 ml) was added and the mixture was heated at 70°C for 15 min. After cooling at room temperature HCl (1 N, 1 ml) was added and the mixture was stirred at 105 °C for 5 min. Sodiumacetate solution (4M, 0.5 ml) was added and the mixture was diluted with water (2.5 ml) and injected onto a semi-preparative HPLC column and 29MBq of the desired product was collected.
HPLC purification: Agilent 1100 series, radioactivity detector: Raytest Gabi; HPLC Column: Zorbax C18 Bonus-RP; 9.2x250; Solvent A: water + 0.1% TFA; Solvent B: acetonitrile:water (9:1) + 0.1 % TFA; Gradient: isocratic 26%B for 2 min then 26% to 41 % B in 20 minutes; Flow: 3 ml/ min.
HPLC analysis: Agilent 1100 series, radioactivity detector: Berthold LB 507B;
HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3);
Gradient: 5%B to 95% B in 7 minutes; Flow: 2 ml/ min. One single radioactive peak as obtained witch co-elutes with the F-19 reference compound.

### 2.4 Synthesis of cold references -N³-(3-(N-(4-[¹⁹F]-fluorobenzoyl))aminopropyl)-O,O-bis-(1'-methoxy-1'-cyclohexyl) thymidine (12) and N³-(3-(N-(4-[¹⁹F]-fluorobenzoyl))amino-propyl)-thymidine (13)

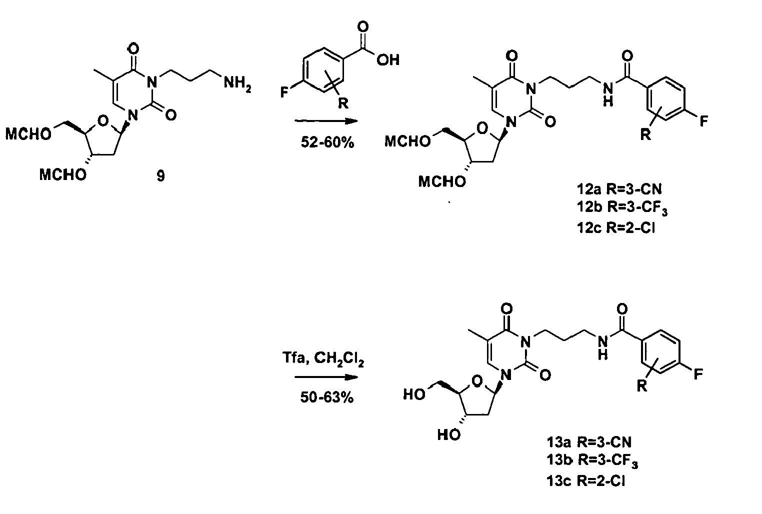

N³-(3-(N-(3-cyano-4-[¹⁹F]-fluorobenzoyl))amino-propyl)-*O*,*O*-bis-(1'-methoxy-1'-cyclohexyl) thymidine 12a,
N³-(3-(N-(3-trifluoromethyl-4-[¹⁹F]-fluorobenzoyl))amino-propyl)-*O*,*O*-bis-(1'-methoxy-1'-cyclohexyl) thymidine 12b and
N³-(3-(N-(2-chloro-4-[¹⁹F]-fluorobenzoyl))amino-propyl)-*O*,*O*-bis-(1'-methoxy-1'-cyclohexyl) thymidine 12c

150 mg (0.29 mmol) compound 9, (0.29 mmol) carbonic acid and 139 mg (0.32 mmol) BOP were solved in 27 ml dichloromethane and cooled down to 0°C. Then 0.074 ml (0.43 mmol) ethyl diisopropyl amine was added. The reaction mixture was stirred at rt for 18h, diluted with dichloromethane, washed with sat. NH₄Cl solution, dried over sodium sulphate, filtrated and concentrated. The residue was purified by chromatography on silica gel to give 52-60% of compound 12.
12a: ¹H-NMR (d-6 DMSO): δ = 8.63 (t, 1 H), 8.29 (dd, 1 H), 8.16 (dd, 1 H), 7.62 (t, 1 H), 7.51 (s, 1 H), 6.14 (t, 1 H), 4.38 (m, 1 H), 3.96 (m, 1 H), 3.84 (t, 2H), 3.45 (t, 2H), 3.22 (m, 2H), 3.06 (s, 3H), 3.04 (s, 3H), 2.16 (m, 2H), 1.80 (s, 3H), 1.74-1.25 (m, 22H) ppm.
12b: ¹H-NMR (d-6 DMSO): δ = 8.69 (t, 1 H), 8.17 (m, 2H), 7.63 (dd, 1 H), 7.51 (s, 1 H), 6.14 (t, 1 H), 4.38 (m, 1 H), 3.97 (m, 1 H), 3.84 (t, 2H), 3.45 (t, 2H), 3.24 (m, 2H), 3.06 (s, 3H), 3.04 (s, 3H), 2.15 (m, 2H), 1.79 (s, 3H), 1.74-1.25 (m, 22H) ppm.
12c: ¹H-NMR (d-6 DMSO): δ = 8.42 (t, 1 H), 7.53 (s, 1 H), 7.47 (m, 2H), 7.26 (m, 1 H), 6.14 (t, 1 H), 4.38 (m, 1 H), 3.97 (m, 1 H), 3.84 (t, 2H), 3.45 (t, 2H), 3.19 (m, 2H), 3.06 (s, 3H), 3.05 (s, 3H), 2.18 (m, 2H), 1.81 (s, 3H), 1.74-1.25 (m, 22H) ppm.
N³'-(3-(N-(3-cyano-4-[¹⁹F]-fluorobenzoyl))amino-propyl)-thymidine (13a)
N³-(3-(N-(4-[¹⁹F]-fluoro-3-trifluormethyl-benzoyl))amino-propyl)-thymidine (13b)
N^{3'}-(3-(N-(2-chloro-4-[¹⁹F]-fluorobenzoyl))amino-propyl)-thymidine (13c)

Compound 12 (0.062 mmol) was solved in 2 ml dichloromethane, cooled down to 0°C and titrated with a solution of trifluoracetic acid in dichloromethane (10%) until complete conversion of the starting material. The reaction mixture was diluted with dichloromethane, washed with bicarbonate and brine, dried over sodium sulphate, filtrated and concentrated. The residue was purified by chromatography on silica gel to give 50-63% of compound 13.
13a: ¹H-NMR (d-6 DMSO): δ = 8.67 (t, 1 H), 8.29 (dd, 1 H), 8.16 (dd, 1 H), 7.73 (s, 1 H), 7.62 (t, 1 H), 6.16 (t, 1 H), 5.24 (d, 1 H), 5.03 (t, 1 H), 4.20 (m, 1 H), 3.83 (m, 2H), 3.73 (s, 1 H), 3.55 (m, 2H), 3.22 (m, 2H), 2.06 (m, 2H), 1.77 (s, 3H), 1.76 (m, 2H) ppm.
13b: ¹H-NMR (CDCl₃): δ = 8.26 (dd, 1 H), 8.11 (m, 1 H), 7.73 (t, 1 H), 7.49 (s, 1 H), 6.26 (t, 1 H), 4.61 (m, 1 H), 4.08 (m, 2H), 4.04 (m, 1 H), 3.94 (dd, 1 H), 3.88 (dd, 1 H), 3.38 (m, 2H), 2.37 (m, 2H), 1.95 (s, 3H), 1.94 (m, 2H) ppm.
13c: ¹H-NMR (d-6 DMSO): δ = 8.46 (t, 1 H), 7.78 (s, 1 H), 7.52 (m, 2H), 7.29 (m, 1 H), 6.23 (t, 1 H), 5.26 (s, 1 H), 5.05 (s, 1 H), 4.26 (m, 1 H), 3.89 (t, 2H), 3.89 (m, 1 H), 3.58 (m, 2H), 3.22 (m, 2H), 2.14 (m, 2H), 1.84 (s, 3H), 1.74 (m, 2H) ppm.

### 3. Synthesis of compound of Formula III by coupling with an alkyl chain

### 3.1 Method

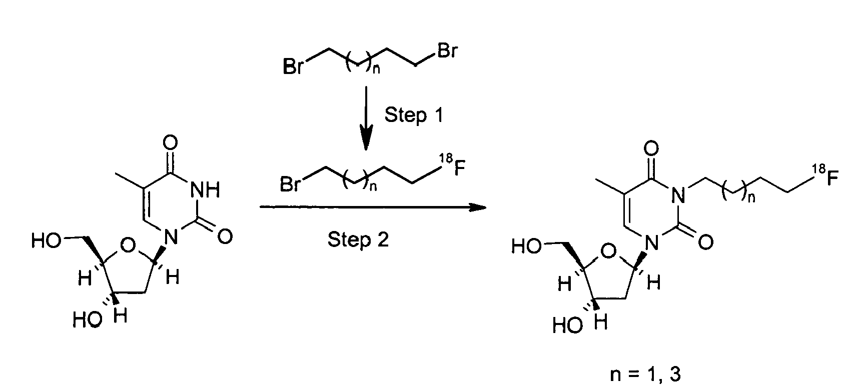

The current method relates to a two step approach for the preparation of 3-(fluoroalkyl) group.
Step 1: Preparation of F-18 fluoroalkyl bromide according to the procedure of Henriksen et.al (J Med Chem. 2005 Dec 1;48(24):7720-32); and Zang et al. (J Med Chem. 2004 Apr 22;47(9):2228-35)
Step 2: Alkylation of thymidine at position 3, was carried out according to the procedure of Al-Madhoun et al. (Mini Rev Med Chem. 2004 May;4(4):341-50)

### 3.2 Synthesis of 3-(4-[¹⁸F]Fluorobutyl)-thymidine (14)

### Step 1 : Radiofluorinations

### Preparation of [¹⁸F]-4-fluorobromobutane:

[¹⁸F]fluoride (1.2 GBq) in 1 mL was trapped on a QMA-cartridge (Waters, Sep Pak Light QMA Part.No.: WAT023525) and eluted using a solution of Kryptofix 2.2.2 (5 mg) and potassium carbonate (1 mg) in aqueous acetonitrile (2 ml; 75 % acetonitrile). The solution was dried by addition and evaporation of anhydrous acetonitrile (3 x 1 mL) under a gentle nitrogen stream at 120 °C. The reaction was cooled to room temperature and to the dried residue was added 1,4-dibromobutane (8.9 mg, 41 µmol) in acetonitrile (250 µl) was added and the mixture was heated at 90 °C for 8 min. The reaction mixture was diluted with water (2 ml) and acetontrile (2 ml) and injected onto a semi-preparative HPLC column and 189MBq of the [¹⁸F]-4-fluorobromobutane was collected. The collected peak was diluted with water (40 ml) and immobilized on a Light tC18 Plus Sep Pak (Part.No.: WAT036810). The Sep Pak was washed with water (5 ml). The Sep Pak was eluted with DMF (5 x 200µl) and the fractions with the [¹⁸F]-4-fluorobromobutane were combined.
HPLC purification: Knauer Typ 64, radioactivity detector: Raytest Gabi; HPLC Column: Zorbax C18 Bonus-RP; 9.4 x 250 mm; Solvent A: water + 0.1% TFA; Solvent B: acetonitrile:water (9:1) + 0.1 % TFA; Gradient: isocratic 55%B for 2 min then 55% to 90% B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min.

### Step 2: Coupling

### Preparation of 3-(4-[¹⁸F]Fluorobutyl)-thymidine:

A solution of [¹⁸F]-4-fluorobromobutane in DMF (48MBq in 400µl) was added to a Wheaton V Vial (5 ml) with thymidine (2mg, 8.3 µmol) and potassium carbonate (2.2 mg, 15.9 µmol). The reaction was stirred at 120 °C for 10 minutes. The reaction was cooled to room temperature, diluted with water (4 ml) and injected onto a semi-preparative HPLC column and 14.7MBq of the 3-(4-[¹⁸F]-Fluorobutyl)-thymidine was collected.
HPLC purification: Knauer Typ 64, radioactivity detector: Raytest Gabi; HPLC Column: Zorbax C18 Bonus-RP; 9.4 x 250 mm; Solvent A: water + 0.1% TFA; Solvent B: acetonitrile:water (9:1) + 0.1 % TFA; Gradient: isocratic 31%B for 2 min then 31% to 41% B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min. One single radioactive peak as obtained (3.4 min) witch co-elutes with the reference compound 3-(4-[¹⁹F]Fluorobutyl)-thymidine described above.

### 3.3 Synthesis of 3-(6-[¹⁸F]Fluoro-hexyl)-thymidine (17)

### Step 1 : Radiofluorinations

### Preparation of [¹⁸F]-6-fluorobromohexane:

[¹⁸F]fluoride (1.28 GBq) in 1 mL was trapped on a QMA-cartridge (Waters, Sep Pak Light QMA Part.No.: WAT023525) and eluted using a solution of Kryptofix 2.2.2 (5 mg) and potassium carbonate (1 mg) in aqueous acetonitrile (2 ml; 75 % acetonitrile). The solution was dried by addition and evaporation of anhydrous acetonitrile (3 x 1 mL) under a gentle nitrogen stream at 120 °C. The reaction was cooled to room temperature and to the dried residue was added 1,4-dibromohexane (8.9 mg, 36 µmol) in acetonitrile (250 µl) was added and the mixture was heated at 90 °C for 8 min. The reaction mixture was diluted with water (2 ml) and acetontrile (2 ml) and injected onto a semi-preparative HPLC column and 320MBq of the [¹⁸F]-6-fluorobromohexane was collected. The collected peak was diluted with water (40 ml) and immobilized on a Light tC18 Plus Sep Pak (Part.No.: WAT036810). The Sep Pak was washed with water (5 ml). The Sep Pak was eluted with DMF (5 x 200µl) and the fractions with the [¹⁸ F]-6-fluorobromohexane in were combined.
HPLC purification: Knauer Typ 64, radioactivity detector: Raytest Gabi; HPLC Column: Zorbax C18 Bonus-RP; 9.4 x 250 mm; Solvent A: water +0.1% TFA; Solvent B: acetonitrile:water (9:1) + 0.1% TFA; Gradient: isocratic 55%B for 2 min then 55% to 90% B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min.

### Step 2 :Coupling

### Preparation of 3-(6-[¹⁸F]Fluoro-hexyl)-thymidine:

A solution of [¹⁸F]-6-fluorobromohexane in DMF (209MBq in 400µl) was added to a Wheaton V Vial (5 ml) with thymidine (2mg, 8.3 µmol) and potassium carbonate (2.2 mg, 15.9 µmol). The reaction was stirred at 120 °C for 10 minutes. The reaction was cooled to room temperature, diluted with water (4 ml) and injected onto a semi-preparative HPLC column and 50MBq of the 3-(6-[¹⁸F]-Fluorohexyl)-thymidine was collected.
HPLC purification: Knauer Typ 64, radioactivity detector: Raytest Gabi; HPLC Column: Zorbax C18 Bonus-RP; 9.4 x 250 mm; Solvent A: water + 0.1 % TFA; Solvent B: acetonitrile:water (9:1) + 0.1% TFA; Gradient: isocratic 31%B for 2 min then 31% to 41% B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min. One single radioactive peak as obtained (3.8 min) witch co-elutes with the reference compound 3-(6-[¹⁹F]-Fluorohexyl)-thymidine.

### 3.4 Synthesis of 3-(4-[¹⁹F]-Fluoro-butyl)-1-[(2R,4S,5R)-4-(1-methoxy-cyclohexyloxy)-5-(1-methoxy-cyclohexyloxymethyl)-tetrahydro-furan-2-yl]-5-methyl-1H-pyrimidine-2,4-dione or 3-(4-[¹⁹F]-Fluorobutyl)-thymidine (18)

40 mg (0.17 mmol) Thymidine, was dissolved in 5ml DMF and 5ml acetone. To this solution was added 46 mg of potassium carbonate (0.33 mmol) and 28 mg of 1-bromo-4-fluorobutane (0.18 mmol) and stirred at room temperature for 72h. Then the reaction mixture was diluted with water, extracted with dichloromethane. The dichloromethane extracts were combined and dried over sodium sulphate. After filtration and removal of the solvent the crude product was purified by preparative HPLC gel to give a quantitative yield of 3-(4-[¹⁹F]-Fluoro-butyl)-thymidine.
HPLC purification: Agilent 1100 series, HPLC Column: Zorbax C18 Bonus-RP; 9.4 x 250 mm; Solvent A: water +0.1% TFA; Solvent B: acetonitrile:water (9:1) + 0.1% TFA; Gradient: 5%B to 95%B in 20 minutes; Flow: 3 ml/ min, UV-Absorptions detector: Agilent 1100 series UV-Detection: 230 nm.
¹H-NMR (d-6 CDCl₃): δ = 7.31 (s, 1 H), 6.17 (t, 1 H), 4.61 (m, 1 H), 4.53 (t, 1 H), 4.41 (t, 1 H), 4.02-3.92 (m, 4H), 3.84 (dd, 1 H), 3.66-3.62 (m, 1H), 2.48-2.44 (m, 1 H), 2.35-2.28 (m, 1 H), 1.93 (s, 3H), 1.77-1.72 (m, 4H) ppm.

### 3.5 Synthesis of 3-(6-[¹⁹F]-Fluoro-hexyl)-1-[(2R,4S,5R)-4-(1-methoxy-cyclohexyloxy)-5-(1-methoxy-cyclohexyloxymethyl)-tetrahydro-furan-2-yl]-5-methyl-1H-pyrimidine-2,4-dione or 3-(6-[¹⁹F]-Fluorohexyl)-thymidine (19)

40 mg (0.17 mmol) Thymidine, was dissolved in 5ml DMF and 5ml acetone. To this solution was added 46 mg of potassium carbonate (0.33 mmol) and 34 mg of 1-bromo-6-fluorohexane (0.18 mmol) and stirred at room temperature for 72h. Then the reaction mixture was diluted with water, extracted with dichloromethane. The dichloromethane extracts were combined and dried over sodium sulphate. After filtration and removal of the solvent the crude product was purified by preparative HPLC gel to give 41,7 mg (73%) of 3-(4-F-19-Fluoro-butyl)-thymidine.
HPLC purification: Agilent 1100 series, HPLC Column: Zorbax C18 Bonus-RP; 9.4 x 250 mm; Solvent A: water + 0.1 % TFA; Solvent B: acetonitrile:water (9:1) + 0.1% TFA; Gradient: 5%B to 95%B in 20 minutes; Flow: 3 ml/ min, UV-Absorptions detector: Agilent 1100 series UV-Detection: 230 nm.
¹H-NMR (d-6 CDCl₃): δ = 7.29 (s, 1 H), 6.16 (t, 1 H), 4.61 (m, 1 H), 4.49 (t, 1 H), 4.37 (t, 1 H), 4.03-4.00 (m, 1 H), 3.96-3.90 (m, 3H), 3.83 (dd, 1 H), 2.50-2.43 (m, 1 H), 2.34-2.27 (m, 1 H), 1.93 (s, 3H), 1.75-1.59 (m, 4H), 1.48-1.35 (m, 4H) ppm.

### 4. Synthesis of compound of Formula III by coupling with

### 4.1 Alkoxyalkyl chain

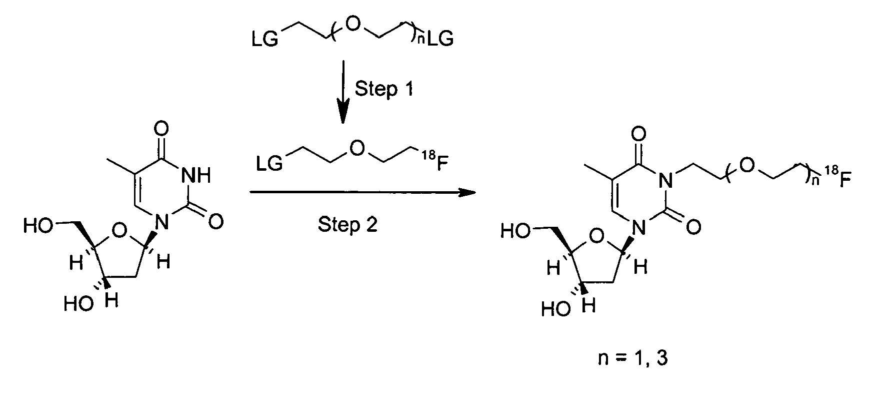

### Step1: Synthesis of [¹⁸F]-(fluoroethyl)(bromoethyl) ether

n=1

[¹⁸F]fluoride (1.13 GBq) in 1 mL was trapped on a QMA-cartridge (Waters, Sep Pak Light QMA Part.No.: WAT023525) and eluted using a solution of Kryptofix 2.2.2 (5 mg) and potassium carbonate (1 mg) in aqueous acetonitrile (2 ml; 75 % acetonitrile). The solution was dried by addition and evaporation of anhydrous acetonitrile (3 x 1 mL) under a gentle nitrogen stream at 120 °C. The reaction was cooled to room temperature and to the dried residue was added bis-(bromoethyl) ether (8.0 mg, Aldrich) in acetonitrile (250 µl) was added and the mixture was heated at 90 °C for 8 min. The reaction mixture was diluted with water (2 ml) and acetontrile (2 ml) and injected onto a semi-preparative HPLC column and 410 MBq of the [¹⁸F]-(fluoroethyl)(bromoethyl) ether was collected. The collected peak was diluted with water (40 ml) and immobilized on a Light tC18 Plus Sep Pak (Part.No.: WAT036810). The Sep Pak was washed with water (5 ml). The Sep Pak was eluted with DMF (5 x 200µl) and the fractions with the [¹⁸F]-(fluoroethyl)(bromoethyl) ether in were combined.
HPLC purification: Knauer Typ 64, radioactivity detector: Raytest Gabi; HPLC Column: Zorbax C18 Bonus-RP; 9.4 x 250 mm; Solvent A: water + 0.1% TFA; Solvent B: acetonitrile:water (9:1) + 0.1% TFA; Gradient: isocratic 35%B for 2 min then 35% to 90% B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min.

### Step 2 :alkylation

### Preparation of N³'-(2-(2-[¹⁸F]Fluoroethoxy)-ethyl)-thymidine (20)

A solution of [¹⁸F]-(fluoroethyl)(bromoethyl) ether in DMF (241 MBq in 400µl) was added to a Wheaton V Vial (5 ml) with thymidine (2mg, 8.3 µmol) and potassium carbonate (2.2 mg, 15.9 µmol). The reaction was stirred at 120 °C for 10 minutes. The reaction was cooled to room temperature, diluted with water (4 ml) and injected onto a semi-preparative HPLC column and 59 MBq of the desired product was collected.
HPLC purification: Knauer Typ 64, radioactivity detector: Raytest Gabi; HPLC Column: Zorbax C18 Bonus-RP; 9.4 x 250 mm; Solvent A: water +0.1% TFA; Solvent B: acetonitrile:water (9:1) + 0.1% TFA; Gradient: isocratic 28%B for 2 min then 28% to 41 % B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min. One single radioactive peak as obtained witch co-elutes with the F-19 reference compound.

### 4.2 Phenylcarbonyl chain

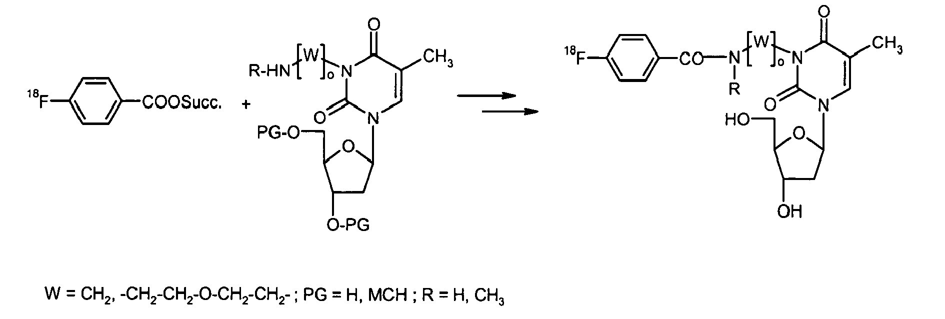

o = 1 to 18 when W = CH₂
o = 1 to 10 when W = CH₂- CH₂-O- CH₂- CH₂
In this method, the radiolabeled coupling agent is ¹⁸F-fluorobenzoic acid succinimidate ester (or other active esters).

### Synthesis of N³-(3-(N-(4-[¹⁸F]-fluorobenzoyl))amino-propyl)-thymidine

PG = MCH
W = (CH₂)₃

N-succinimidyl 4-[¹⁸F]fluorobenzoate was prepared according to a known literature procedure (Appl Radiat lsot. (2003), 59:43-8). This prosthetic group (255 MBq) was coupled to compound 9 as reported in an analogues way (Eur J Nucl Med Mol Imaging 31:1081-1089; Nucl Med Biol 31:179-89.): A solution of N-succinimidyl 4-[¹⁸F]fluorobenzoate in DMF (255 MBq in 400µl) was added to a Wheaton V Vial (5 ml) with thymidine derivative 9 (2mg). The reaction was stirred at 80 °C for 10 minutes. The reaction was cooled to room temperature. TFA (1 ml) was added, the reaction mixture was stirred for 10 min at 80°C, diluted with water (4 ml) and injected onto a semi-preparative HPLC column and 22.7MBq of the desired product was collected.
HPLC purification: Knauer Typ 64, radioactivity detector: Raytest Gabi;_ HPLC Column: Zorbax C18 Bonus-RP; 9.4 x 250 mm; Solvent A: water +0.1% TFA;
Solvent B: acetonitrile:water (9:1) + 0.1% TFA; Gradient: isocratic 25%B for 2 min then 25% to 41 % B in 20 minutes; Flow: 3 ml/ min.
HPLC analysis: Agilent 1100 series, detector: Berthold LB 507B; HPLC Column: ACE 3µ C18 Bonus-RP; 4.6 x 50 mm; Solvent A: 10mM K₂HPO₄ in water; Solvent B: 10mM K₂HPO₄ in acetonitrile:water (7:3); Gradient: 5%B to 95% B in 7 minutes; Flow: 2 ml/ min. One single radioactive peak as obtained witch co-elutes with the F-19 reference compound.

### 5. Test for In Vitro Phosphorylation Activity:

A very important requirement for successful usage of proliferation imaging, is the mono-phosphorylation of the thymidine compounds. This is the first step in providing the cell with thymidine triphosphates, which the cell needs for DNA-synthesis, the primary stage of the cell cycle leading in the end to a doubling of the cell. Mono-phosphorylation is, at the same time, necessary for the retention of compounds within the cell to allow for an accumulation leading to the necessary compound concentration. The enzyme catalyzing the monophosphorylation is the thymidine kinase (EC 2.7.1.21). An in vitro phosphotransfer assay with rekombinante thymidine kinase was established to evaluate the substrate quality of the compounds. This assay was conducted in 50 mM Tris-HCl, ph 7.6, 0.5 mM MgCl₂, 100 mM KCl, 0.5 mg/ml BSA, 100 µM ATP + P-32-ATP (0.7 mCi/mmol) plus 10-60 ng thymidine kinase. The P-32 labeled products were separate by TLC and quantitated on a phosphoimager.

### 6. Test for In Vitro Metabolic Stability:

Metabolic stability is a prerequisite for successful accumulation, since the compounds have to reach their target without being degraded or modified to allow uptake into the cells and phosphorylation by thymidine kinase. It has been shown already extensively that endogene thymidine or its exogene analogues are mainly degraded by one enzyme, thymidine phosphorylase (EC 2.1.1.45). This enzyme utilizes a phosphate to convert thymidine into thymine and 2-deoxyribose 1 phosphate. In the serum of humans the concentration of thymidine phosphorylase is high, so almost all thymidine is degraded and hardly any thymidine can be detected in human serum. Thymidine analogues which are resistant to degradation by thymidine phosphorylase are quite stable in serum, indicating that it is thymidine phosphorylase which is mostly responsible for the degradation. The activity of thymidine phosphorylase can also be measured in vitro by an spectrophotometric assay. Here recombinant thymidine phosphorylase is incubate in 200 mM PBS, ph 7.4 containing 1 mM thymidine or thymidine analogue. The conversion can be measured by following an absorption change at 290 nm in the cuvette.

### 7. Test for In Vitro Label Stability:

Metabolic stability if the compound as well as its label is important for allowing enrichment in proliferating cells, since the compounds have to reach their target still labeled without being degraded or modified to allow uptake into the cells. In order to test for in vitro stability of the compound as well as the label, the labeled compounds are incubated in heparinized human serum at 37°C. At various time points aliquods are taken and serum proteins are precipitated by the addition of acetonitrile, pelleted by centrifugation and the supernatant is analyzed by HPLC for metabolites and intact label.

## Claims

1. A compound according to formula (I) 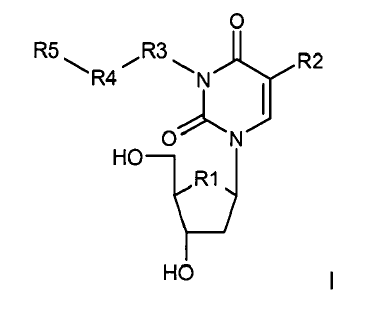 wherein
R1 is O, S, CH₂, C(=CH₂), C=O, C=S, NH or N-alkyl;
R2 is H, linear or branched alkyl, CF₃, Cl, Br or I;
R3 is selected from
a) [alkoxyl]ₙ-alkyl;
b) higher alkyl;
c) benzoyl-alkyl; and
d) benzoyl
wherein n is 1 to 10;
R4 is a linker;
R5 is a radioisotope label,
and pharmaceutically acceptable salt, hydrate or solvate thereof.

2. The compound according to claim 1 wherein R3 is a benzoyl.

3. The compound according to claim 1 wherein R3 is a higher alkyl.

4. The compound according to claim 3 wherein the higher alkyl is a C₈ to C₁₈ alkyl.

5. The compound according to claims 3 and 4 wherein the higher alkyl is a C₈ to C₁₂ alkyl.

6. The compound according to claim 1 wherein R3 is [alkoxyl]ₙ-alkyl
wherein n is 1 to 10.

7. The compound according to claim 6 wherein [alkoxyl]ₙ-alkyl is selected from
a) -(CH₂-CH₂-O)ₘ-(CH₂-CH₂)-;
b) -(CH₂-CH₂-CH₂O)-(CH₂-CH₂-O)ₘ-(CH₂-CH₂)-; and
c) -(CH₂-CH₂-CH₂O)-(CH₂-CH₂-O)ₘ-(CH₂-CH₂-CH₂)-wherein m is 1 to 6.

8. The compound according to claim 1 wherein the R3 is benzoyl-alkyl.

9. The compound according to any preceding claims wherein
R1 is selected from O, S and C(=CH₂).

10. The compound according to any preceding claims wherein
R2 is a C₁ to C₄ alkyl.

11. The compound according to any preceding claims wherein the linker R4 is a bond or Aryl-L
wherein L is selected from
a) -C(O)N(H);
b) -C((O)N(Me);
c) -SO₂-N(H)-; and
d) -SO₂-N(Me)-;
and wherein Aryl is an aromatic moiety optionally substituted with:
a) Hydrogen;
b) Halo;
c) Cyano;
d) Nitro;
e) Trifluormethyl;
f) -S(O)₂-methyl;
g) -S(O)₂-ethyl;
h) -C(O)-Me or
a combination thereof.

12. The compound according to claim 11 wherein independently aromatic moiety is a phenyl and L is selected from -C(O)N(H) and -SO₂-N(Me)-.

13. The compound according to any preceding claims wherein the radioisotope label R₅ is selected from ¹⁸F , ⁷⁷Br, ⁷⁶Br, ¹²³I, ¹²⁵I, and ¹¹C.

14. The compound according to any preceding claims wherein the radioisotope label R₅ is ¹⁸F.

15. The compound according to any preceding claims
N³-(2-(2-[¹⁸F]Fluoroethoxy)-ethyl)-*O,O*-bis-(1'-methoxy-1'-cyclohexyl) thymidine 15,
N³-(3-(N-(4-[¹⁸F]-fluoro-3-cyano-benzoyl))aminopropyl)-thymidine 16,
N³-(2-(2-[¹⁸F]Fluoroethoxy)-ethyl)-thymidine 20,
N³-(3-(N-(4-[¹⁸F]-fluoro-3-cyano-benzoyl))aminopropyl)-thymidine 13aa,
N³-(3-(N-(4-[¹⁸F]-fluoro-3-trifluoromethyl-benzoyl))aminopropyl)-thymidine 13bb,
N³-(3-(N-(4-[¹⁸F]-fluoro-2-chloro-benzoyl))aminopropyl)-thymidine 13cc and
N'-(3-(N-(4-[¹⁸F]-fluorobenzoyl))amino-propyl)-thymidine.

16. The compound according to claims 1 to 15 for use as diagnostic imaging agent.

17. The compound according to claims 1 to 15 for use as diagnostic imaging agent for positron emission tomography (PET).

18. The compound according to claims 1 to 15 for use as medicament.

19. A composition comprising a compound according to claims 1 to 15 and a pharmaceutical acceptable carrier or diluent.

20. Use of a compound according to claims 1 to 15 for the manufacture of diagnostic imaging agent.

21. The use according to claim 20 for the manufacture of diagnostic imaging agent for imaging tissue proliferations, hyperplastic inflammation, benign tumours or malignant tumours.

22. Use of a compound according to claims 1 to 15 for the manufacture of medicament.

23. The use according to claim 22 for the manufacture of medicament for treating proliferative diseases.

24. The use according to claim 23 wherein proliferative disease is a disease developing malignant tumor selected from malignant lymphoma, pharyngeal cancer, lung cancer, liver cancer, bladder tumor, rectal cancer, prostatic cancer, uterine cancer, ovarian cancer, breast cancer, brain tumor, and malignant melanoma.

25. A compound according to formula (II) 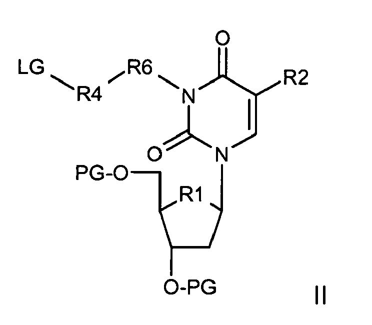 wherein
PG is selected from:
a) hydrogen;
b) SiR⁷_{3;}
c) CH₂-Z;
d) C(O)-Q;
e) C(O)O-E;
f) -(R⁸-phenyl);
g) -((R⁸)₂-phenyl);
h) (2-tetrahydropyranyl;
i) (1-alkoxy)-alkyl;
j) (1-alkoxy)-cycloalkyl;
k) allyl; and
l) *tert*-butyl;
Z is selected from
a) phenyl;
b) alkoxy;
c) benzyloxy;
d) triphenyl;
e) (methoxyphenyl)phenyl;
f) di(methoxyphenyl)phenyl;
g) α-naphtyldiphenyl;
h) hydrogen; and
i) methylsulfanyl;
Q is selected from
a) hydrogen;
b) C₁-C₅ linear or branched alkyl;
c) halomethyl;
d) dihalomethyl;
e) trihalomethyl;
f) phenyl;
g) biphenyl;
h) triphenylmethoxymethyl; and
i) phenoxymethyl;
E is selected from
a) lower linear or branched alkyl;
b) methoxymethyl;
c) vinyl;
d) allyl;
e) benzyl;
f) methoxyphenyl;
g) dimethoxyphenyl; and
h) nitrophenyl;
R⁷ is independently from each other selected from
a) lower linear or branched alkyl;
b) phenyl; and
c) benzyl;
R⁸ is selected from methoxy and halo;
LG is a leaving group;
R1, R2 and R4 are defined as in the preceding claims 1 to 12; and
R6 is selected from
a) [alkoxyl]ₙ-alkyl
b) C₁-C₁₈ alkyl;
c) benzoyl-alkyl; and
d) benzoyl;
wherein n is 1 to 10,
and pharmaceutically acceptable salt, hydrate or solvate thereof.

26. The compound according to claim 25 wherein PG is (1-alkoxy)-cycloalkyl

27. The compound according to claims 25 and 26 wherein the leaving group (LG) is selected from
1) NO₂, (CH₃)₃N⁺ if R4 is aryl for radiofluorination;
2) (alkyl)₃Sn if R4 is aryl for radioiodination and ¹¹C-methyliodide (Stille-cross-coupling), and
3) Cl, Br , I, OTs, OMs, OTf, ONs if R4 is bond for radiofluorination.
4) hydroxy if R4 is aryl for ¹¹C alkylation:

28. The compound according to claims 25 and 27 wherein R6 is C₈-C₁₈ alkyl.

29. The compound according to claim 25 as followed
3-t-butyldimethylsilyloxybutyl-4-(1-methoxy-cyclohexyloxy)-5(1-methoxy-cyclohexyloxy-methyl)-thymidine 2^{a},
3-t-butyldimethylsilyloxyhexyl-4-(1-methoxy-cyclohexyloxy)-5(1-methoxy-cyclohexyloxy-methyl)-thymidine 2b,
3-(4-Hydroxybutyl)-4'-(1-methoxy-cyclohexyloxy)-5'(1-methoxy-cyclohexyloxymethyl)-thymidine 3a,
3-(6-Hydroxyhexyl)-4'-(1-methoxy-cyclohexyloxy)-5'-(1-methoxy-cyclohexyloxymethyl)-thymidine 3b,
3-(4-O-Tosyl)butyl -4'-(1-methoxy-cyclohexyloxy)-5'-(1-methoxy-cyclohexyloxymethyl)-thymidine 4a,
3-(6-O-Tosyl)hexyl)-4'-(1-methoxy-cyclohexyloxy)-5'-(1-methoxy-cyclohexyloxymethyl)-thymidine 4b,
3-(4-O-Tosyl)butyl-thymidine 5a,
3-(6-O-Tosyl)hexyl-thymidine 5b,
3-(4-O-methanesulfonyl)butyl-4'-(1-methoxy-cyclohexyloxy)-5'-(1-methoxy-cyclohexyloxymethyl)-thymidine 6a,
3-(6-O-methanesulfonyl))hexyl)-4'-(1-methoxy-cyclohexyloxy)-5'-(1-methoxy-cyclohexyloxymethyl)-thymidine 6b,
3-(4-O-methanesulfonyl)butyl-thymidine 7a,
3-(6-O- methanesulfonyl)hexyl-thymidine 7b,
3-(3-(4-trimethylaminobenzoyl)amino)propyl-4'-(1-methoxy-cyclohexyloxy)-5'-(1-methoxy-cyclohexyloxymethyl)-thymidine 10 and 3-(3-(4-trimethylaminobenzoyl)amino)propyl-thymidine 11.

30. A method for obtaining compound of formula (III) 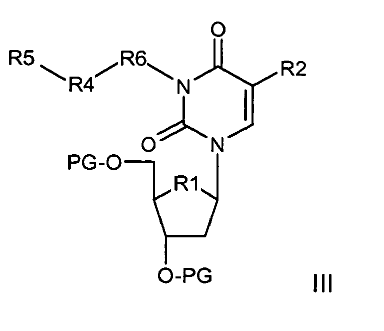 comprising the step of reacting compound of formula (II) 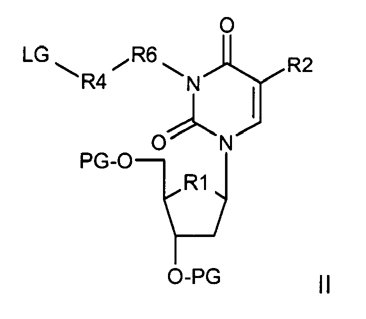 wherein
PG and LG are defined as in preceding claims 25 and 26 and R1, R2, and R4 are defined as in preceding claims 1 to 12;
R6 is selected from
a) [alkoxyl]ₙ-alkyl
b) C₁-C₁₈ alkyl;
c) benzoyl-alkyl and
d) benzoyl
wherein n is 1 to 10
with R5 that is defined as in preceding claims 1, 13 and 14;
and thereafter converting the compound of formula III into a pharmaceutically acceptable salt, hydrate or solvate thereof if desired.

31. A method for obtaining compound of formula (III) 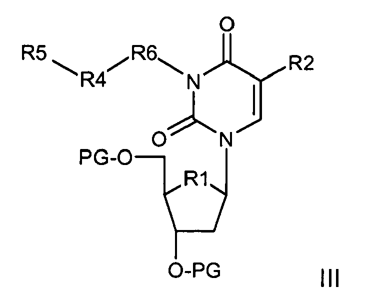 comprising the steps of coupling compound of formula (IV) 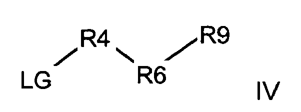 wherein
LG is a leaving group,
R9 is selected from iodo, bromo, chloro, mesyloxy, tosyloxy, trifluormethylsulfonyloxy and nonafluorobutylsulfonyloxy,
R4 is defined as in preceding claims 1 and 2
R6 is selected from
e) [alkoxyl]ₙ-alkyl
f) C₁-C₁₈ alkyl;
g) benzoyl-alkyl and
h) benzoyl
wherein n is 1 to 10
with R5 that is defined as in preceding claims 1, 13 and 14, for obtaining a single radiolabeled compound (IV) then reacting the resultant labeled compound (IV) with a compound of formula (V) 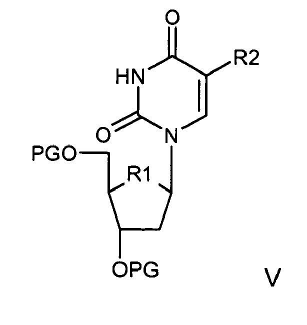 wherein PG, R1 and R2 are defined as in preceding claims 1 to 12. and thereafter converting the compound of formula III into a pharmaceutically acceptable salt, hydrate or solvate thereof if desired.

32. The method according to claims 30 and 31 wherein R6 is C₈-C₁₈ alkyl.

33. The method according to claims 30 to 32 wherein the radioisotope label R5 is ¹⁸F.

34. A kit comprising
a) compound of formula I;
b) compound of formula II;
c) compound of formula III; or
d) compound of formula IV and V.

35. A kit according to claim 33 for imaging tissue proliferations, hyperplastic inflammation, benign tumours or malignant tumours.

36. A method for diagnosing tissue proliferations, hyperplastic inflammation, benign tumours or malignant tumours comprising the steps of
a) administering to a patient a compound of formula I, and
b) measuring positron emission by PET.

37. The method according to claim 35 wherein the diagnosis is a diagnosis concerning localization, progress or determination of therapeutic effect of tissues proliferation, hyperplastic inflammation or benign or malignant tumours.

38. Compound selected from
N³-(3-(N-(4-[¹⁹F]-fluorobenzoyl))amino-propyl)-*O*,*O*-bis-(1'-methoxy-1'-cyclohexyl) thymidine 12,
N³-(3-(N-(4-[¹⁹F]-fluorobenzoyl))amino-propyl)-thymidine 13, N³-(3-(N-(3-cyano-4-[¹⁹F]-fluorobenzoyl))amino-propyl)-*O*,*O*-bis-(1'-methoxy-1'-cyclohexyl) thymidine 12a,
N³-(3-(N-(3-trifluoromethyl-4-[¹⁹F]-fluorobenzoyl))amino-propyl)-*O*, *O-*bis-(1'-methoxy-1'-cyclohexyl) thymidine 12b,
N³-(3-(N-(2-chloro-4-[¹⁹F]-fluorobenzoyl))amino-propyl)-*O*, *O*-bis-(1'-methoxy-1'-cyclohexyl) thymidine 12c,
N³-(3-(N-(3-cyano-4-[¹⁹F]-fluorobenzoyl))amino-propyl)-thymidine 13a,
N³-(3-(N-(4-[¹⁹F]-fluoro-3-trifluormethyl-benzoyl))amino-propyl)-thymidine 13b,
N³-(3-(N-(2-chloro-4-[¹⁹F]-fluorobenzoyl))amino-propyl)-thymidine 13c,
3-(4-[¹⁹F]-Fluorobutyl)-thymidine 18,
3-(6-[¹⁹F]-Fluorohexyl)-thymidine 19,
3-(4-[¹⁸F]-Fluorobutyl)-thymidine 14, and
3-(6-[¹⁸F]-Fluorohexyl)-thymidine 17.
